# EUROPEAN PATENT SPECIFICATION

(11) **EP 1 771 555 B1**
(45) Date of publication and mention of the grant of the patent: **12.01.2011**
(21) Application number: 05784046.4
(22) Date of filing: 29.07.2005
(51) Int. Cl.: C12N 9/22

(54) **METHOD FOR PRODUCING HIGHLY SENSITIVE ENDONUCLEASES, NOVEL PREPARATIONS OF ENDONUCLEASES AND USES THEREOF**
VERFAHREN ZUR HERSTELLUNG VON HOCHEMPFINDLICHEN ENDONUKLEASEN, NEUARTIGE ZUBEREITUNGEN VON NUKLEASEN UND IHRE VERWENDUNG
PROCEDE DE PRODUCTION D'ENDONUCLEASES A SENSIBILITE ELEVEE, NOUVELLES PREPARATIONS D'ENDONUCLEASES ET LEURS UTILISATIONS

(30) Priority: 30.07.2004 WO PCT/EP2004/009166; 30.07.2004 WO PCT/EP2004/009159
(43) Date of publication of application: 11.04.2007
(73) Proprietor: Genoplante-Valor, 75015 Paris (FR); INSTITUT NATIONAL DE LA RECHERCHE AGRONOMIQUE (INRA), 75007 Paris (FR)
(72) Inventor: BENDAHMANE, Abdelhafid, F-91830 LE COUDRAY MONTCEAUX (FR); STURBOIS, Bénédicte, F-91130 RIS ORANGIS (FR); TRIQUES-ROSTAING, Karine, F-75013 PARIS (FR); CABOCHE, Michel, F-78310 MAUREPAS (FR)
(74) Representative: Vialle-Presles, Marie José
(86) International application number: PCT/EP2005/009220
(87) International publication number: WO 2006/010646

(56) References cited:
- WO-A-01/62974
- WO-A-03/066809
- WO-A-2004/035771
- OLEYKOWSKI C.A. ET AL.: "MUTATION DETECTION USING A NOVEL PLANT ENDONUCLEASE" NUCLEIC ACIDS RESEARCH, OXFORD UNIVERSITY PRESS, SURREY, GB, vol. 26, no. 20, 1998, pages 4597-4602, XP002943289 ISSN: 0305-1048 cited in the application
- FISCHER R. ET AL.: "TOWARDS MOLECULAR FARMING IN THE FUTURE: TRANSIENT PROTEIN EXPRESSION IN PLANTS" BIOTECHNOLOGY AND APPLIED BIOCHEMISTRY, ACADEMIC PRESS, US, vol. 30, October 1999 (1999-10), pages 113-116, XP000923028 ISSN: 0885-4513
- SHURVINTON CLAIRE E ET AL: "A nuclear localization signal and the C-terminal omega sequence in the Agrobacterium tumefaciens VirD2 endonuclease are important for tumor formation" PROCEEDINGS OF THE NATIONAL ACADEMY OF SCIENCES OF USA, NATIONAL ACADEMY OF SCIENCE, WASHINGTON, DC, US, vol. 89, no. 24, 1992, pages 11837-11841, XP002159986 ISSN: 0027-8424
- DATABASE BIOSIS [Online] BIOSCIENCES INFORMATION SERVICE, PHILADELPHIA, PA, US; February 2002 (2002-02), TRIFONOVA E A ET AL: "Transgenic tobacco (Nicotiana tabacum SR1) plants expressing the gene coding for Serratia marcescens nuclease." XP002356380 Database accession no. PREV200400054982 & GENETIKA, vol. 38, no. 2, February 2002 (2002-02), pages 274-277, ISSN: 0016-6758

## Description

The invention relates to the identification and preparation of mismatch specific endonucleases having a high activity and sensitivity and a broad substrate specificity.

In the beginning of the last century, the discovery of the possibility to induce mutations within the DNA by radiations or chemicals has brought a considerable hope to understand the gene function *in vivo.* Since then, mutagenesis and natural sequence variation have been widely used to identify new functions, genes corresponding to a specific function as well as active sites within a specific protein.

A critical aspect in implementing this approach, in particular in the case of point mutations, is the choice of mutation detection methods that are designed to screen large stretches of DNA without reducing diagnostic sensitivity or specificity, while at the same time providing information about the location of the mutation. Among the most used tools are methods based on imperfectly matched DNA that could be created *in vitro* by denaturation and renaturation of two DNA molecules. Mismatches are detected in these heteroduplexe molecules using chemicals like groove binders or molecules that can cleave specifically single strand DNA at the mismatch site. Alternatively, single strand specific endonucleases have been used to cleave the DNA at the mismatch site. Most of the endonucleases described this far belong to S1/P1 class of nucleases.

Nucleases such as S1, P1 and mung bean nuclease, belonging to a same family designated as: "S1/P1 nucleases family", or as: "S1 nucleases family" are known to cut DNA at regions of single-strandedness. These nucleases, however, have acid pH optima in the range of 4.0 - 5.0.

This is disadvantageous for mismatch detection, since low pH values favour DNA depurination and destabilizes DNA duplexes, leading to non-specific DNA degradation, and reducing the sensitivity and specificity of the detection.

A few years ago, OLEYKOWSKI et al. (Nucleic Acids Res, 26, 4597-4602, 1998) detected in extracts from various plants a mismatch endonuclease activity having a neutral pH optimum (around pH 8) and performing a single-strand cut on the 3'side of a mismatch site. These authors reported that this mismatch endonuclease activity was associated with mannosyl glycoproteins in extracts of alfalfa sprout, asparagus, celery and tomato. The enzyme from celery, named CEL I, was purified from celery stalks by successive steps of ammonium sulfate precipitation, binding to a concanavalin A-agarose column and elution by [alpha]-d+-mannose, binding to a phosphocellulose column and elution by a linear gradient of KCI, and fractionation by size exclusion chromatography. The preparation of CEL I thus obtained contained several protein bands of 34-39 kDa.

YANG et al., (Biochemistry, 39, 3533-3541, 2000), and PCT application WO 01/62974 describe an improved purification of CEL I by use of alphamethyl-mannoside in the purification buffers to overcome the aggregation of CEL I with endogenous lectins. These documents also disclose the cloning of CEL I cDNA. On the basis of sequence data, CEL I was assigned to a sub-family of the S1/P1 nucleases family, and several potential homologues encoded by the genes BFN1 of Arabidopsis (GenBank nucleotide AY040016), ZEN1 of Zinnia (GenBank (nucleotide) AB003131), and DSA6 of daylily (GenBank (nucleotide) AF082031) were identified.

CEL I endonuclease activity has been shown to be highly specific for base-substitution mismatches and for mismatches resulting from insertion/deletion events, and to be independent of the flanking sequence context. It is thus useful as a mutation detecting reagent in various methods involving mutational screening. The CEL I mismatch detection system is a simple assay that requires PCR amplification of the target sequence, denaturation and annealing to allow formation of heteroduplexes between the wild type and the mutant allele, enzymatic mismatch cleavage, and analysis of the product by gel electrophoresis. It is advantageous over other popular mismatch detection systems, like denaturing HPLC, because of its specificity and sensitivity for detection of mismatches in large stretches of DNA.

By way of example, OLEYKOWSKI et al. and YANG et al. (publications cited above) report its use to detect sequence alterations in the human *BRCA1* gene, and SOKURENKO et al. (Nucl. Acids Res., 29, e111, 2001) disclose its use to detect mutations and polymorphisms in large regions of genomic DNA. CEL I is also used for high-throughput screening in TILLING (Targeting Induced Local Lesions IN Genomes), in which chemical mutagenesis is followed by screening for point mutations, or for detection of polymorphisms in natural populations, also called "Ecotilling" (COMAÏ et al, Plant Journal, 37, 778-786, 2004). It has been used in plants (COLBERT et al., Plant Physiology 126, 480-484, 2001; TILL et al., Genome Research 13, 524-530, 2003; PERRY et al., Plant Physiology 131, 866-871, 2003) as well as in animals such as zebrafish (WIENHOLDS et al., Genome Res., 13, 2700-2707, 2003). PCT application WO 03/066809 proposes to use CEL I in a method for reassorting sequence variations among related polynucleotides, named "Genetic Reassortment by Mismatch Resolution" (GRAMMR).

CEL I has however the disadvantage of having an efficiency of cleavage that varies from one mismatch to another: in the case of a DNA loop with a single nucleotide insertion, OLEYKOWSKI et al. (Nucleic Acids Res. 1998 Oct 15;26(20):4597-602) report that CEL I substrate preference is G>A>C>T; in the case of base-substitution mismatches, CEL I substrate preference is C/C ≥ C/A ∼ C/T ≥ G/G > A/C ∼ A/A ∼ T/C > T/G ∼ G/T ∼ G/A ∼ A/G > T/T. Its efficiency is significative on the mismatches C/A, C/C, C/T, G/G. A decrease of the activity is observed for the others, and it is almost inefficient on the mismatch T/T. This variation in the efficiency of cleavage may result in a lower accuracy in the detection of some mutations when the detection of one allele in a pool of DNA is required.

Another inconvenient limiting the use of CEL I is the low yield of the available purification methods. OLEYKOWSKI et al., starting with 7 kg of celery stalk containing about 350 g protein obtained 3 ml CEL I at 0.1 µg/µl; The purification procedure disclosed by YANG et al. and in PCT WO 01/62974 results in 5µg of purified CEL I with a specific activity of 3.1 x 10⁷ CEL I units/mg protein starting from 10⁵ kg of celery stalk.

It has been proposed, in order to obtain larger amounts of CEL I, to produce it by recombinant DNA technology. PCT application WO 03/066809 proposes a large list of potentially suitable vectors and host cells including almost any known prokaryotic or eukaryotic expression systems; however, the only expression system actually disclosed in this document is a tobamovirus-based vector. The construct resulting from cloning in said vector of the cDNA of CEL I fused to a sequence encoding a 6-Histidine tag has been used to infect tobacco plants. Recombinant CEL I was recovered from intracellular fluid of the infected plants and purified by metal affinity chromatography on nickel-NTA resin. PCT WO 03/066809 is silent about the yield of purified enzyme. It indicates that its activity in a GRAMMR reaction is similar to the one of the native enzyme purified from celery. One of the inconveniences of this system is that viruses tend to recombine, loosing part or completely the expressed gene. This leads to the production of truncated forms in addition of the full-length CEL I, decreasing the specific activity of the enzyme.

PCT Application WO2004/035771 relates to a method for producing CEL I in yeast. To this effect, a synthetic gene encoding CEL I was constructed by modifying the native DNA sequence of CEL I according to codon usage in yeast. This document indicates that the recombinant CEL I produced by this synthetic gene is able to recognize all possible mismatch combinations, and exemplifies the recognition and cleavage of the mismatch A/A. On the other hand, it is silent about the mismatch preference of said recombinant CEL I.

It appears from the above that the currently available methods for producing recombinant CEL I do not provide any significant improvement over the production of native CEL I from celery. Further they do not address the problem of the substrate preference of native CEL I.

Also, it would be desirable to have other endonucleases able, like CEL I, to cleave single base pair mismatches in heteroduplex DNA templates under neutral pH, but which have a different mismatch preference, or preferably, that cleave equally well all mismatches. However, such endonucleases have not been identified until now.

The existence of CEL I-like endonuclease activities has been reported in many plants (cf. for instance OLEYKOWSKI et al., 1998 cited above). However, the enzymes responsible for these activities have not been characterized, their biochemical properties, such as substrate preference have not been studied, and their sequences have not been identified. On the other hand, structural homologues of CEL I have been identified *in silico* (YANG et al., cited above; TILL et al. Nucleic Acids Res., 32, 2632-41, 2004). Three of them (BFN1 of Arabidopsis, ZEN1 of Zinnia, and DSA6 of daylily) have been reported to be involved in plant senescence (PEREZ-AMADOR et al., Plant Physiol. 122, 169-180 2000). However, they have not been purified and remain uncharacterised biochemically: in particular, their efficiency to recognise mismatches in heteroduplex DNA *in vitro* has not been tested. An endonuclease called SP, that has been assigned to the S1/P1 family on the basis of its activity, and that has a neutral pH optimum has been purified from spinach. Like CEL I, this enzyme cleaves insertion/deletions and base-substitution mismatches; however it does not recognize those containing a guanine residue (OLEYKOWSKI et al. Biochemistry, 38, 2200-5, 1999).

Seeking for alternative methods to obtain recombinant CEL I, the inventors have tried to express it *in planta* via *Agrobacterium* mediated transient expression.

They have expressed recombinant CEL I in agroinfiltrated tobacco leaves, and have purified it from the leave extract by ammonium sulphate precipitation. They have found that, surprisingly, they obtained a very high yield of recombinant CEL I with a high activity, and furthermore, that said recombinant CEL I preparation recognises the mismatches with a broader specificity and a higher sensitivity than the preparations of CEL I known in the prior art, allowing a clear detection even of mismatches, such as T/T, which were deemed as poorly recognized by CEL I.

In view of these results, the inventors have had the idea to use this method for screening endonucleases identified *in silico* as belonging to the S1/P1 family, by testing their activity *in vitro.*

The instant invention thus provides a simple and rapid method to obtain great quantities of endonucleases, in particular S1/P1 nucleases, from a small quantity of starting material.

A mismatch specific endonuclease is defined herein as an endonuclease which is able to cleave specifically all the base-substitution mismatches (*i*.*e*., A/A, G/G, C/C, T/T, A/G, A/C, G/T, C/T, G/A, C/A, T/C, T/G), as well as insertion/deletions of one or more nucleotides.

An object of the present invention is thus a method for producing a mismatch-specific recombinant endonuclease, wherein said method comprises:
- expressing said recombinant endonuclease in cells of a whole host plant or of an organ detached therefrom transiently transformed by agroinfiltration with an *Agrobacterium* strain containing an expression vector comprising a polynucleotide encoding said endonuclease;
- isolating said recombinant endonuclease from said host plant cells.

Said plant cells may be part of a cell suspension, or of a tissue or organ culture. In this case, the enzyme can be collected from the supernatant and/or from the cultured cells or tissue or organ. Preferably, they will be part of a whole plant or of an organ detached therefrom; in this case, the transient transformation with the *Agrobacterium* strain will be performed by agroinfiltration.

Agroinfiltration is a transient expression method based on the delivery of Agrobacteria containing a gene of interest into intact plant tissue.

A DNA construct comprising a gene of interest is cloned into a binary vector and transferred into a chosen *Agrobacterium* strain, and the transformed Agrobacteria are grown to log phase or to saturation and collected in the same way as for conventional *Agrobacterium* mediated transformation. Classically, agroinfiltration is performed by applying a suspension of the transformed *Agrobacterium* cells either by injection into an organ (generally the leaves) of the chosen plant using a syringe without a needle, or by vacuum infiltration for a few minutes. After release of the vacuum, the organ, or the entire plant is placed in a growth chamber. The expressed protein of interest is extracted from the infiltrated organ, usually one to four days post-infiltration. Agroinfiltration protocols are disclosed in various publications, for instance, KAPILA et al., (Plant Sci.122, 101-108, 1997); BENDAHMANE et al., (Plant Cell, 11, 781-792, 1999); SCOFIELD et al., (Science. , 274, 2063-5, 1996); TANG et al., (Science., 274, 2060-3, 1996); MARILLONNET et al., (Proc Natl Acad Sci U S A, 101, 6852-7, 2004); WROBLEWSKI et al., (Plant Biotech. J., 3, pp. 259-273,2005).

The classical protocols used for *Agrobacterium* mediated transient expression, and in particular for agroinfiltration can be used in the practice of the present invention. A large choice of *Agrobacterium* strains, of binary vectors, and of regulatory elements controlling the expression of the gene of interest, is available, and one of skill in the art can choose among them the more appropriate, for instance according to the host plant that one intends to use.

In the experimentations disclosed in the Examples below, the inventors have used a pBIN19-derived binary vector, pBIN61, and agrobacterium strain C58C1 harboring the hypervirulence pCH32 plasmid, and the cDNA or the genomic coding sequences have been expressed under CaMV 35S promoter. However, other binary vectors, other strains of *Agrobacterium* and other constitutive or inducible promoters can be used with the same result.

Advantageously, agroinfiltration will be performed in the leaves, which can optionally be detached from said plant immediately before, or immediately after, the infiltration.

Host plants that can be used in the method of the invention include any plant that is compatible with *Agrobacterium* transformation. Preferred plants include in particular those of the genus *Nicotiana,* in particular *Nicotiana benthamiana* and *Nicotiana tabacum.*

According to a preferred embodiment of the invention, said endonuclease is isolated from an agroinfiltrated plant organ, in particular an agroinfiltrated leaf by a process comprising the following steps:
- extracting the cell content from the agroinfiltrated organ expressing said endonuclease;
- adding ammonium sulfate at a final concentration of 30 % or more to said extract, and separating the protein precipitate from the supernatant;
- adding ammonium sulfate at a final concentration of 80 % or more to said supernatant, and recovering the protein precipitate containing the endonuclease.

Said protein precipitate is resuspended in an appropriate buffer, for instance a buffer comprising Tris HCl (pH 8), PMSF and 10 % glycerol. It can be used directly, or stored at -80°C until use.

Alternatively, the total extract obtained after the first step indicated above can be used as such, without performing precipitation steps with ammonium sulfate.

Optionally, the endonucleases produced by the method of the invention can be further purified, by any appropriate method known in itself, such as column affinity purification where CEL I is tagged with a tag that has an affinity to a specific component in the column. According to a preferred embodiment, CEL I of the invention is provided with a 6-Histidine tag, and purified by metal affinity chromatography on nickel-NTA.

A method for testing whether a candidate endonuclease is a mismatch-specific endonuclease comprises:
a) producing said candidate endonuclease under recombinant form by the method of the invention, as defined above;
b) testing said recombinant endonuclease for its ability to degrade single stranded DNA;
c) testing said recombinant endonuclease for its ability to cleave a test heteroduplex DNA fragment at a pre-defined mismatch site;
d) testing said recombinant endonuclease for its ability to cleave heteroduplex DNA fragments carrying all the types of mismatches (*i*.*e*., the base substitution mismatches A/A, G/G, C/C, T/T, A/G, A/C, G/T, C/T, G/A, C/A, T/C, T/G, as well as insertion or deletion mismatches)

If an endonuclease passes the tests of steps b) c) and d) (*i.e.,* if it is able to degrade single stranded DNA, to cleave a test heteroduplex DNA fragment at a pre-defined mismatch site, and to cleave heteroduplex DNA fragments carrying all the types of mismatches), it is considered as a mismatch-specific endonuclease.

A method for screening mismatch-specific endonucleases comprises:
a) producing candidate endonucleases under recombinant form by the method of the invention, as defined above;
b) testing said recombinant endonucleases for their ability to degrade single stranded DNA;
c) testing the recombinant endonucleases able to degrade single stranded DNA, and testing them for their ability to cleave a test heteroduplex DNA fragment at a known and well characterized mismatch site;
d) selecting the recombinant endonucleases able to cleave a test heteroduplex DNA fragment at a known and well characterized mismatch site, and testing them for their ability to cleave heteroduplex DNA fragments carrying all the types of mismatches.
e) selecting the recombinant endonucleases that pass the tests of steps b) c) and d).

The above defined methods can further comprise a step consisting of testing said endonuclease(s) for its (their) sensitivity by testing their ability to detect a mutant allele in a DNA pool, in presence of an excess of the wild-type allele, and selecting the endonucleases that are able to detect said mutant allele in the presence of at least 9-fold excess of the wild-type allele *(i.e.,* one mutant allele in a pool of 10), preferably in the presence of at least 14-fold excess, still more preferably in the presence of at least 19-fold excess, and by order of increasing preference, 29-fold excess, 39-fold excess, 49-fold excess, 59-fold excess of the wild-type allele.

Preferably, the above defined tests are performed in a reaction mixture having a pH from 7 to 8, advantageously from 7.4 to 7.8, and containing from 5 to 20 mM, advantageously 10 mM MgCl₂. Advantageously, said reaction mixture also comprises from 0.5 mM to 2 mM, and preferably 1mM DTT. The inventors have also observed that addition of PEG-8000, at from 2% to 10% (w/v), and in particular 5% of the final reaction mixture, increased the global activity of the endonucleases.

The candidate endonucleases that can be tested by the above defined methods can be found among those of the S1/P1 family. In the PFAM database (BATEMAN et al., Nucleic Acids Res. 32, D138-41, 2004), this family is designated as PFAM 02265. One can for instance use the profile HMM (Hidden Markov Models) built from PFAM 02265 as a probe to screen the available DNA sequence data banks, using the HMMER software, to identify candidate endonucleases in different plants.

Alternatively, the InterPro IPR003154 code (corresponding to S1/P1 nucleases) can be used for screening the content of databases, for example using the following address:
http://www.ebi.ac.uk/interpro/ISpy?ipr=IPR003154

Screening the Trembl/Swissprot database with this code identified 43 proteins (Table 1).

**Table 1: result of the screening of the Trembl/Swissprot database with the IPR003154 code.**

| Accession number Trembl or Swissprot | Description | Seq Length | Bit (Blastp) |
|---|---|---|---|
| P24504 | Nuclease PA3 (EC 3.1.3.6) | 270 | 127 |
| P24289 | Nuclease P1(EC 3.1.30.1) | 270 | 127 |
| P24021 | Nuclease S1 (EC 3.1.30.1) | 267 | 107 |
| Q00235 | Nuclease S1 precursor. | 287 | 109 |
| Q9P356 | Nuclease Le1. | 310 | 111 |
| Q7S8Q5 | Hypothetical protein. | 306 | 114 |
| Q87OT1 | Probable nuclease S1. | 324 | 119 |
| Q8NIH8 | Nuclease Le3. | 298 | 129 |
| Q25267 | 3'-nucleotidase/nuclease. | 477 | |
| Q9GNZ4 | 3'-nucleotidase/nuclease precursor. | 378 | |
| Q9NJ13 | 3'-nucleotidase/nuclease. | 377 | |
| Q9NJY3 | Single strand-specific nuclease. | 315 | |
| Q86GJ3 | P4 nuclease. | 316 | |
| Q8T4M4 | Class I nuclease. | 3i6 | |
| 065424 | Putative bifunctional nuclease | 362 | 217 |
| 065425 | Putative bifunctional nuclease | 454 | 164 |
| 080326 | Endonuclease precursor. | 303 | 480 |
| 081656 | Senescence-associated protein 6. | 298 | 461 |
| 081958 | Endonuclease. | 288 | 289 |
| Q93WW9 | Si-type endonuclease (Fragment). | 1361 | 239 |
| Q9ARD4 | Putative nuclease. | 289 | |
| Q9C9G4 | Putative bifunctional nuclease | 290 | 300 |
| Q9FTRO | Putative bifunctional nuclease. | 310 | |
| Q9FTR1 | Putative bifunctional nuclease. | 311 | 180 |
| Q9LGA5 | ESTs D48949(5i554i). | 3081 | 293 |
| Q9LL59 | CEL I mismatch endonuclease. | 296 | 627 |
| Q9SXG1 | Nuclease I. | 290 | 291 |
| Q9ZR87 | Bifunctional nuclease. | 328 | 308 |
| Q9ZR88 | Bifunctional nuclease (Fragment). | 280 | 305 |
| Q9ZR89 | Bifunctional nuclease bfnl. | 305 | 445 |
| Q7XND5 | 0SJNBbOO34l13.4 protein. | 252 | 277 |
| Q7XPN4 | OSJNBaOO6ODO6.10 protein. | 290 | 275 |
| Q8LA68 | Endonuclease, putative. | 296 | 281 |
| Q8LCL6 | Putative bifunctional nuclease. | 290 | 299 |
| Q8LDW6 | Putative bifunctional nuclease. | 294 | 274 |
| 068530 | Endonuclease S1 homolog. | 309 | |
| Q8XRE8 | putative signal peptide protein. | 337 | |
| Q989R8 | Endonuclease. | 278 | |
| Q7P202 | Probable endonuclease. | 274 | |
| Q8F378 | Nuclease Si (EC 3.1.30.1). | 306 | |
| Q8P5Y5 | Endonuclease. | 270 | |
| Q8PHA3 | Endonuclease. | 271 | |
| Q9SXA6 | Bifunctional nuclease bfnl. | 305 | 448 |

This analysis is preferably completed by performing a Blast on the databases (blastp or tblastn), using a reference protein sequence (for example the CEL I sequence), and selecting the best hits.

The examples below will describe with more details the testing of five candidate endonucleases from *Arabidopsis thaliana.*

The inventors found that one of these endonucleases, which is represented in the annexed sequence listing under SEQ ID NO: 2 (the corresponding DNA sequence is represented under SEQ ID NO: 1), and which corresponds to BFN1 of *Arabidopsis thaliana,* has a different specificity, and a far greater sensitivity than CEL I.

This discovery of the properties of BFN1 as a mismatch-specific endonuclease, which were unknown until now, allows to propose its use as a mutation detecting reagent, for detecting mismatches resulting from base substitutions, as well as from insertion/deletions of one or more nucleotides.

BFN1, as well as any mismatch-specific endonucleases that can be identified according to the method of the invention can be obtained in great quantities by the method of production of endonucleases.

The present invention also encompasses recombinant endonuclease preparations obtainable by the method of production according to the invention. These are in particular recombinant CEL I preparations and recombinant BFN1 preparations.

The recombinant CEL I preparations of the invention have a different mismatch specificity and a higher sensitivity than the CEL I preparations of the prior art. The recombinant CEL I preparations of the invention, have the following mismatch preference: T/G ∼ A/G ∼ G/G ∼ G/T ∼ T/T ∼ G/A ≥A/A ∼ C/C ≥T/C ∼ C/T > A/C ∼ C/A, while the mismatch preference of the CEL I preparations of the prior art is C/C ≥ C/A ∼ C/T ≥ G/G > A/C ∼ A/A ∼ T/C > T/G ∼ G/T ∼ G/A ∼ A/G > T/T. The recombinant CEL I preparations of the invention can recognize a mutant allele in the presence of a 23-fold excess of the wild type allele, while the CEL I preparations of the prior art do not efficiently recognize a mutant allele when diluted over a 8-fold dilution.

The recombinant BFN1 preparations of the invention have also a different mismatch specificity than both the CEL I preparations of the prior art and the recombinant CEL I preparations of the invention. The recombinant BFN1 preparations of the invention, have the following mismatch preference: G/G ∼ G/A ∼ A/G ∼ G/T ∼ T/G > T/T ∼ A/A ∼ C/C ∼ T/C > C/T ∼ A/C ∼ C/A. The mismatch preferences for each of these enzymes are summarized in Table 2 below.

**Table 2: comparison of mismatch recognition preferences.**

| Protein | Most efficiently recognized | | Well recognized | | | Recognized with a lower efficiency | | | Very weakly recognized |
|---|---|---|---|---|---|---|---|---|---|
| CEL I from Celery (prior art) | C/C | C/T | G/G | A/C | | A/A | G/A | G/T | T/T |
| | C/A | | | | | T/C | T/G | A/G | |
| Recombiant CEL I according to the invention* | T/G | A/G | T/T | G/A | A/A | C/T | A/C | T/C | |
| | G/G | G/T | C/C | | | C/A | | | |
| BFN1 (ENDO1) | G/G | G/A | A/A | T/T | T/C | C/T | A/C | C/A* | |
| | A/G | G/T | C/C | | | | | | |
| | T/G | | | | | | | | |

| | | | | | | | | | |
|---|---|---|---|---|---|---|---|---|---|
| * = these mismatches are recognized more efficiently by BFN1 (ENDO1) than by recombinant Cel I. ** N.B.: the estimation of the cleavage efficiency is only semi-quantitative; therefore, slight variations can occur in the ordering of the mismatches, based on their recognition by the endonucleases, depending on the investigator and/or the experiment. | | | | | | | | | |

The recombinant BFN1 preparations of the invention further have a higher sensitivity than both the CEL I preparations of the prior art and the recombinant CEL I preparations of the invention. The recombinant BFN1 preparations of the invention can recognize a mutant allele in the presence of a 59-fold excess of the wild type-allele.

The recombinant BFN1 or CEL I endonuclease preparations of the invention can be used, as a mutation detecting reagent, in any method involving mismatch screening, as mentioned above. They are particularly advantageous in genotyping, in TILLING, High-throughput TILLING, Ecotilling, GRAMMR, etc.

Methods for using the endonuclease preparations of the invention involve basically the same steps as those of the prior art, *i*.*e*., PCR amplification of the target sequence, denaturation of the amplification product and annealing to allow formation of heteroduplexes between the wild type and the mutant allele, cleavage of the heteroduplexes by the endonuclease, and analysis of the cleavage products. A mix of different endonucleases can advantageously be used for cleaving the heteroduplexes when performing these methods.

Due to their high sensitivity, the endonuclease preparations of the invention also enable to perform high-throughput methods for identifying mutations in a sample. For example, endonuclease preparations according to the invention can be used to perform methods such as described in WO 01/75167, with a far larger number of samples, since it is possible to pool many samples together for analysis.

According to the invention, the endonuclease preparations described above can be used to screen one or more mutations in a target gene, in a large number of samples from any organism or cell-line derived therefrom, by performing the following steps:
a) amplifying said target gene or a part thereof from each individual of said population,
b) ordering said amplification products in a 2- or 3-dimensional matrix, comprising lines, rows (2-D matrix) and columns (3-D matrix),
c) pooling said amplification products such as to obtain different pools, each pool representing a row, a line or a column of said matrix,
d) adding to each pool a reference amplification product obtained from a non-mutated gene, and incubating such pools in conditions enabling the formation of heteroduplexes,
e) incubating each pool with an endonuclease preparation according to the invention, and
f) detecting the presence of heteroduplexes in said incubated pools.

Alternatively, the above method can be performed by first ordering the samples in the matrix, then pooling them (with addition of the reference), and performing the amplification, incubation and detection steps.

Depending on the numbers of samples to screen, the matrix can be a 2 or 3-D matrix. For example, if 576 samples are to be screened, a 24 x 24 matrix can be used. If 13824 samples are to be screened, a 3-D matrix may thus be more appropriate (24 x 24 x 24). Only 72 reactions would be needed to screen this population, and the mutated genes would be individualized by the column, row and line pools to which it belongs.

The reference amplification product corresponds to an amplification product obtained from the reference gene (compared to which mutations are searched), amplified with the same primers as the target gene in the population. It is important to add a reference amplification product. Indeed, although it is very unlikely that all samples harbour the exact same mutation as compared to the reference gene, this will ensure that heteroduplexes may be formed if the pool contains a target gene that harbours a mutation.

The present invention will be further illustrated by the additional description which follows, which refers to examples illustrating the preparation and properties of recombinant CEL-I endonuclease, the testing of five candidate endonucleases from *Arabidopsis thaliana,* and the identification of BFN1, (hereinafter also designated as ENDO1) as a mismatch specific endonuclease. It should be understood however that these examples are given only by way of illustration of the invention and do not constitute in any way a limitation thereof.

### LEGENDS TO THE FIGURES:

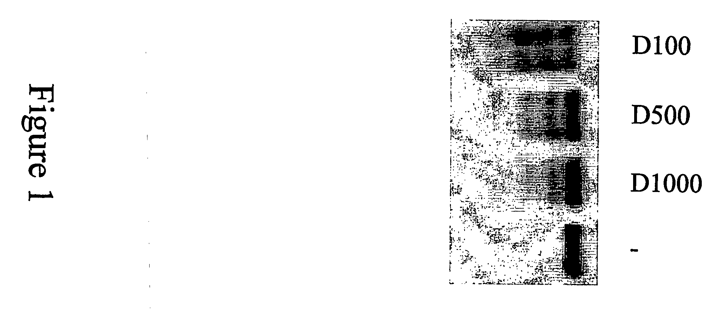

**Figure 1****:** Detection of point mutation on agarose gel. Heteroduplexes (of wild-type and mutant DNAs) have been incubated with different dilutions of recombinant Cel I (D104 to D1000), or without protein (-).

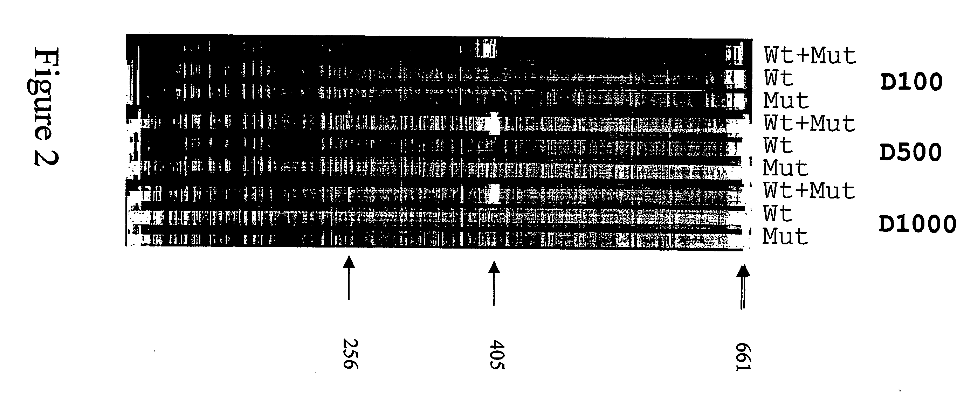

**Figure 2****:** Detection of point mutation on acrylamide gel. WT+ Mut : DNA from the wild-type and the mutant have been mixed together before the PCR, thereby generating heteroduplexes. WT: only wild-type DNA has been used for the PCR, thereby generating only homoduplexes. Mut : only mutant DNA has been used for the PCR, thereby generating only homoduplexes. D100, D500 and D 1000: dilutions of the recombinant protein Cel I.
   On the top of the gel is indicated the homoduplexes size (661 bp). The arrow at 405 bp shows the fragment labeled with the FAM fluorochrome. The arrow at 256 bp shows the fragment labeled with the ROX fluorochrome.

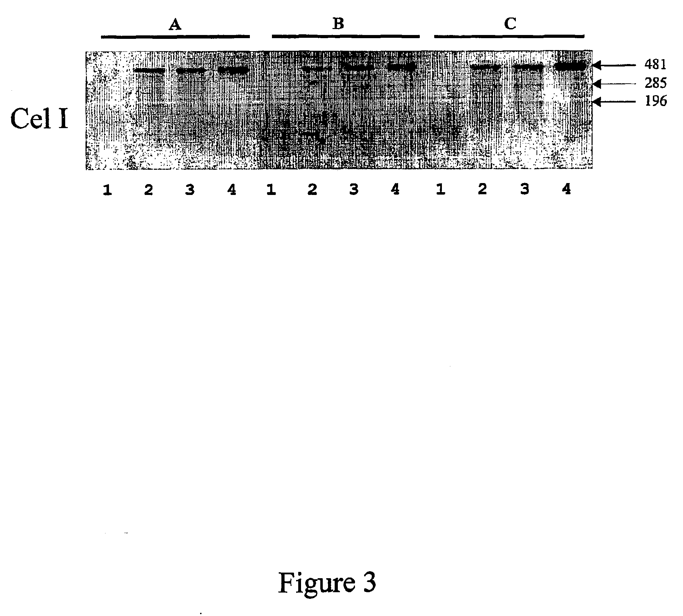

**Figure 3**: Detection of point mutation in genomic DNA on agarose gel. PCR products were obtained as disclosed in Example 4 below, with primers 4-960 and 4-721 (SEQ ID Nos: 3 and 4). They were then digested with different dilutions of recombinant CEL I preparation obtained by ammonium sulfate precipitation (as disclosed in Example 2), and analysed on agarose gel. The size of the PCR products for the rms 1-13 mutant and wild-type alleles, is around 500 bp (481bp exactly). As a result of the detection of the mutation in heteroduplex DNA, two bands of approximatively 200 and 300 bp were obtained. The two bands can be seen in an agarose gel even at a dilution of 1/1000 of the protein produced in tobacco.
   1: D100; 2: D500; 3: D1000; 4: no enzyme.
   A: Terese; B: rms1.13; C: T+rms1.13.

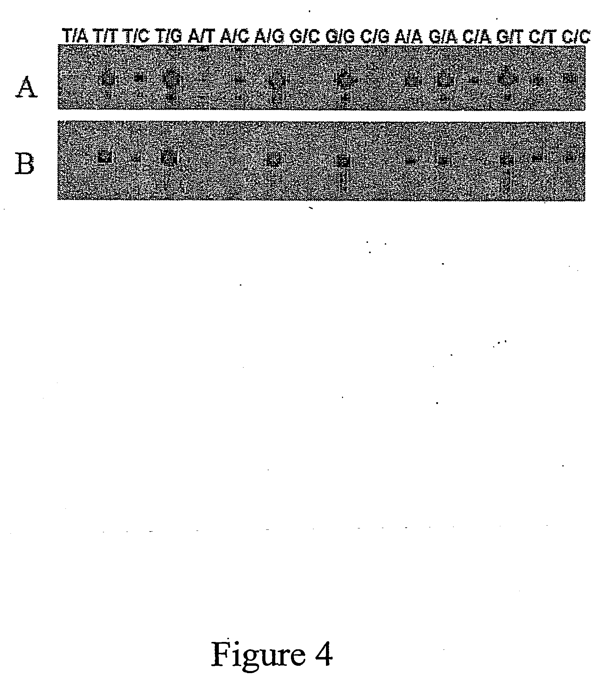

**Figure 4****:** Analysis of recombinant Cel I activity on all types of mismatches. A series of mutants based on the Rx gene were created by PCR, and heteroduplexes were obtained by mixing the amplification products corresponding to those different mutants. Labeled oligonucleotides with IRD700 and IRD800 fluorophore (MWS®) were used for the PCR, and mismatch detection was performed on LICOR4300 (LICOR ®). The figure shows IRD dye 700 (A) and IRD dye 800 (B) channels of a run. The channel A shows the 204 bp fragment with the 5'end fragment IRD-700-labeled and the channel B shows the 438 bp fragment labeled on 3'end fragment with IRD-800 dye.

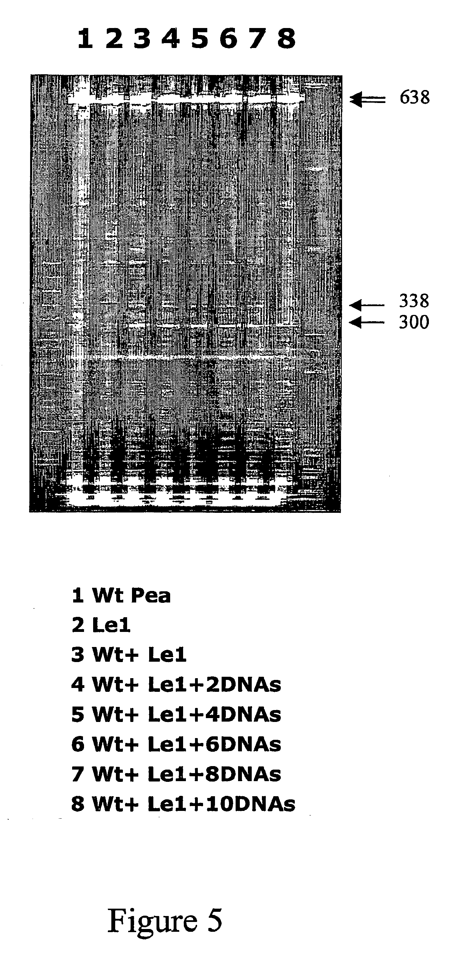

**Figure 5****:** Sensitivity of the recombinant Cel I protein produced in tobacco. 1 : Wt Pea; 2 : Le1 ; 3 Wt+ Le1 ; 4 Wt+ Le1+2DNAs ; 5 Wt+ Le1+4DNAs ; 6 Wt+ Le1+6DNAs ; 7 Wt+ Le1+8DNAs ; 8 Wt+ Le1+10DNAs.

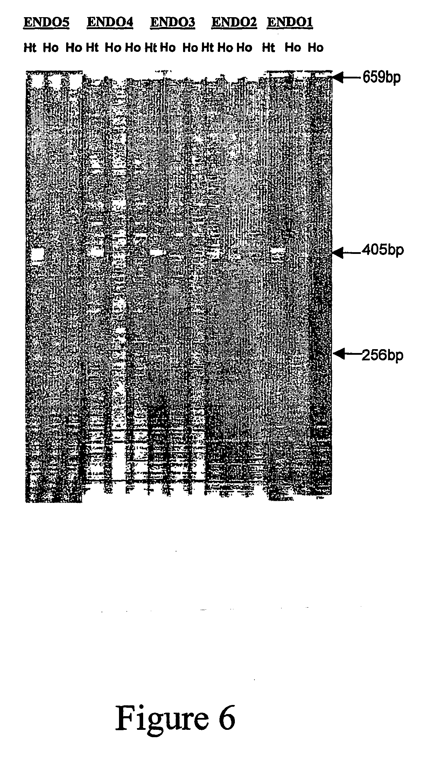

**Figure 6**: Cleavage of heteroduplex DNA at C-A/T-G mismatch site by five candidate endonucleases. (Ho)= homoduplex DNA ; (Ht) = heteroduplex DNA.

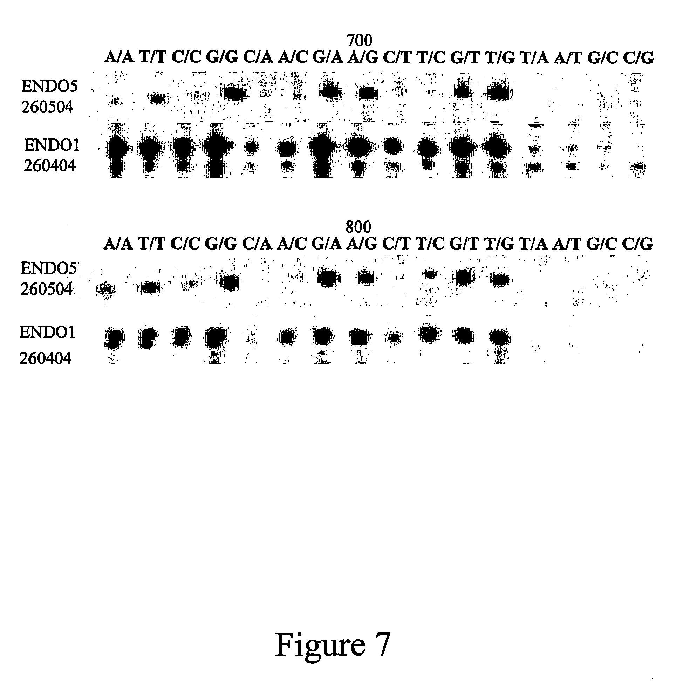

**Figure 7**: Detailed analysis of the specific recognition of all the types of mismatches with ENDO1 and ENDO5. The same protocol as for figure 4 has been used.

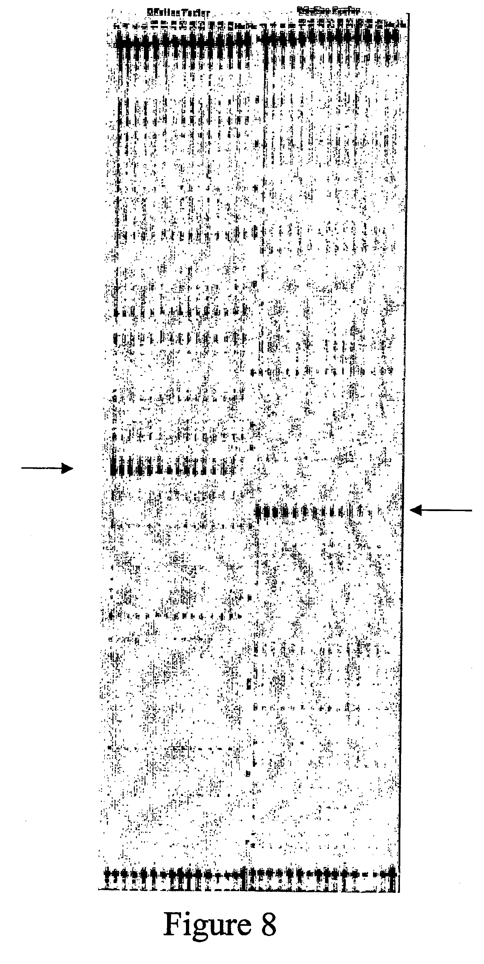

**Figure 8****:** Test for measuring the detection sensitivity of ENDO1. A mixture of mutant (Le-1) and wild-type (Torstag) DNAs has been used as template for ENDO1 activity, in the following ratios: 1 mutant for 2, 4, 6, 8, 10, 15, 20, 25, 30, 35, 40, 50, and 60 wild-type (from left to right). The two last lines of each panel are homoduplexes (only mutant and only wild-type). Left panel: IRD700 channel, size of the detected fragment = 338bp. Right panel: IRD800 channel, size of the detected fragment = 300bp.

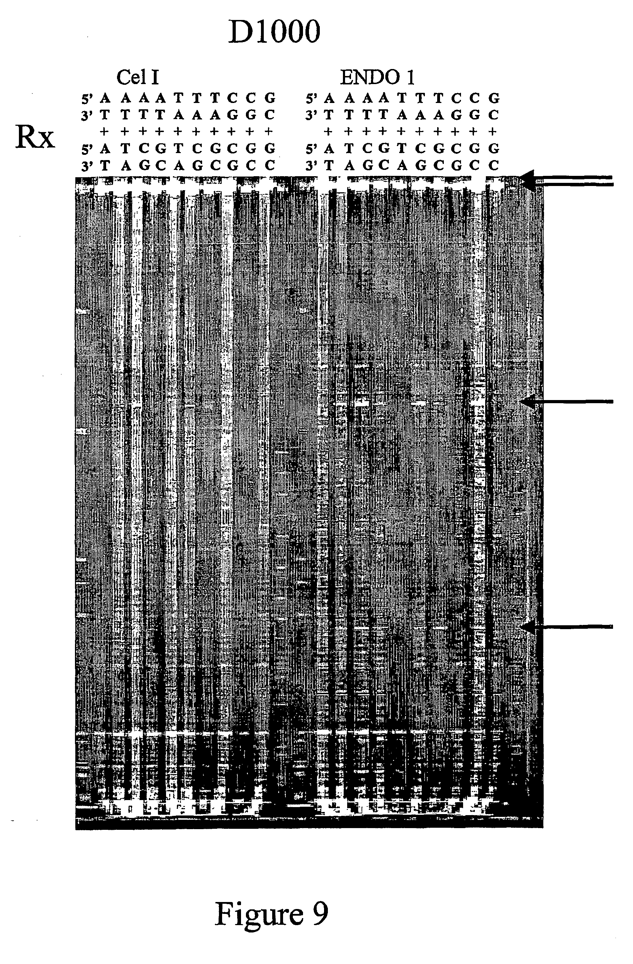

**Figure 9****:** Comparison of mismatches detection by Cel1 and Endo1. Lines 1, 5, 8 and 10 correspond to homoduplexes. Fragments resulting from the cleavage by the endonuclease are of 405 bp (labelled in blue) and 256 bp (labelled in red, less visible in black and white). Endo1 recognizes mismatches more efficiently than Cell. Moreover, the background (non-specific activity) is far lower with Endo1.

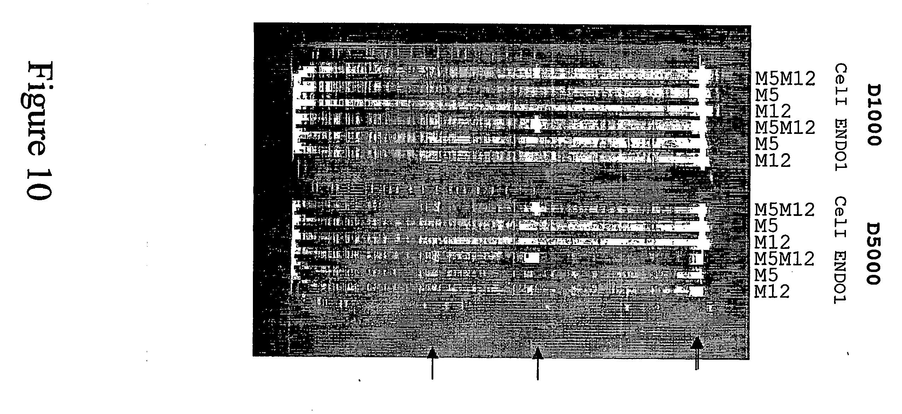

**Figure 10****:** Detection of a known point mutation on acrylamide gel with ENDO1 in two different dilutions (D1000 and D5000). M5 and M12 are the two plasmids containing the Rx gene; one contains the Wt form and the other one the mutated form. Primers : Rx21rox and Rx22fam (in this figure, the smaller cleavage band, labelled in red, appeared clearly on the gel but is less visible in black and white).

### EXAMPLE 1: CLONING, PURIFICATION AND PRODUCTION OF CEL I ENDONUCLEASE

### Cloning of the cDNA of CEL I

### Extraction of RNA from celery

Young leaf tissues (1 g) were ground in liquid nitrogen using a pestle and mortar. The powder was suspended in 10 ml Trizol (Gibco) extraction buffer. The suspension was mixed with 2 ml of chloroform and centrifuged at 12 000 rpm at 4 °C for 15 min. The supernatant was mixed with an equal volume of isopropanol and total RNA were precipitated by centrifugation at 12 000 rpm for 10 minutes at 4°C. The pellet was washed with 80% ethanol, air-dried and resuspended in 200µl of DEPC water.

### DNase treatment

DNase treatment was carried out to hydrolyse DNA contaminating the RNA preparation. 10 µg of total RNA were incubated with 10 units of DNase following the manufacturer conditions (Promega).

The reaction was incubated at room temperature for 15 minutes then stopped by addition of EDTA to 25mM final concentration and by heat inactivation of the DNase at 65°C for 10 minutes.

### CEL I cDNA synthesis

Ten microliters of DNAse treated-RNA was used for first strand cDNA synthesis. First strand cDNA synthesis was primed with 2 picomoles of 20 mers oligo dT primer. The reaction mix of 50 µl (10 µl of 5x Superscript buffer [GIBCO-BRL], 5 µl of 100 mM DTT, 5 µl of 5 mM dNTP) was heated at 70 °C for 10 min and then cooled on ice. 1 µl of Superscript reverse transcriptase (200 units/µl; GIBCO-BRL) and 1 µl RNase inhibitor (37 units/µl; Pharmacia) were added and the reaction was incubated at 42 °C for 1 hour. PCR amplification was used to convert the first strand cDNA into double strand DNA by PCR amplification using two primers specific to 5' and 3' UTR of CEL I (see Table 3 below).

### Cloning of CEL I and expression in tobacco leaves

Full length CEL I open reading frame was PCR amplified and inserted between the 35S promoter and the transcriptional terminator of CaMV in the binary vector pBin19 to create pBIN35S-CELI. Another construct pBIN35S-CELI8His was also constructed. pBIN35S-CELI8His is identical to pBIN35S-CELI except that a Histidine tag of 8 amino acids was inserted at C-terminal of CEL I protein. The oligonucleotides used to create pBIN35S-CELI and pBIN35S-CELI8His are indicated in table 3 below.

**TABLE 3:**

| Primer name | |
|---|---|
| CEL N terminal | 5' TATCGTTCTAGAGGGAATGACGCGATTATATTCTGTGTTC 3' SEQ ID N° 5 |
| CEL C terminal | 5' TATCTGAATTCATGCCAAAGAATGATC 3' SEQ ID N°6 |
| CELC terminal 8 His | 5'AATTCAATGGTGATGGTGGTGATGGTGATGTGCCAAAGAATGATCTGCGG 3' SEQ ID N°7 |

These constructs were transformed into Agrobacterium strain C58C1 carrying the virulence helper plasmid pCH32 (Hamilton et al. PNAS, 93(18) : 9975-9979, 1996). pCH32 expresses VirG and VirE and was used to enhance T-DNA transfer. Agrobacterium cells were inoculated into 5 mL of L broth medium (Sambrook et al. 1989) supplemented with 50 µg/mL kanamycin and 5 µg/mL tetracycline and grown at 28°C overnight. L broth medium (50 mL) supplemented with 50 µg/mL kanamycine, 5 µg/mL tetracycline was then inoculated with the 5-mL overnight cultures and grown at 29°C for 2 days. Cells were precipitated and resuspended to a final concentration of 0.5 OD_{6 00} in a solution containing 10 mM MgCl2, 10 mM MES, pH 5.6, and 150µM acetosyringone. The cultures were incubated at room temperature for 2 hr before agroinfiltration into *Nicotiana benthamiana* leaves.

The Agrobacterium suspension was injected in the leaves using a syringe without needle.

Agroinfiltrated *Nicotiana benthamiana* plants were incubated for at least 48 hours at 24°C, 16 hours of light, 60 % of humidity. To test for the efficiency of agroinfiltration, plants were also agroinfiltrated with a construct expressing the green fluorescent protein (GFP). Before harvesting the leaves, the intensity of the expression of the GFP for each leaf was checked using an UV lamp. The plant leaves were harvested only if the GFP was expressed.

### Preparation of the protein from tobacco leaves by ammonium sulfate precipitation

Agroinfiltrated tobacco leaves were harvested and weighted. Two grams of agroinfiltrated leaves were homogenized in 7 ml of buffer containing 0.1M Tris-HCl pH8, 200µM PMSF, 0.125mM β-mercaptoethanol and 10% of glycerol and then centrifuged at 3000 g for 25 minutes to pellet the cellular debris. To the supernatant 100% ammonium sulfate was added to the final concentration of 30 %, and the samples were then incubated on ice for 1 hour, and centrifuged at 30 000g for

30 minutes at 4°C to pellet proteins that precipitate at 30% ammonium sulfate. To the supernatant 100% ammonium sulfate was added to obtain 80 % final concentration and the samples were again incubated on ice for 1 hour and centrifuged as above to pellet the proteins that precipitate at 80% ammonium sulfate. The pellet containing the proteins precipitated at 80% of ammonium sulfate was resuspended in 600µl of homogeneization buffer. The protein concentration was determined by the kit Coomassie Plus Reagent Assay (Pierce). The homogeneized pellet contains 14µg protein/µl. Thus, 8400µg proteins were recovered from 2 grams of agroinfiltrated tobacco leaves. The homogeneized pellet was diluted at 1µg/µl in a buffer containing 50 mM Tris-HCl pH8, 10% glycerol and 100µM PMSF, aliquoted and stored at - 80°C.

### Purification of the His-6 tagged protein by Ni-NTA affinity chromatography:

Five grams of agroinfiltrated tobacco leaves were collected and homogenized in 15 ml iced buffer containing Na-Phosphate (100mM), Tris HCl pH 8 (10mM), NaCl (200mM), Sodium methabisulfite (0.2%), PMSF (1mM), βMercaptoethanol (10mM). After homogenization, the sample was centrifugated at 6000 g (Beckman, rotor JA 20) for 10 minutes at 4°C. Imidazole (10mM) was added to the supernatant and the pH was ajusted to 9 with NaOH. The solution was centrifugated at 42 000 g (Beckman, rotor JA 20) for 60 minutes at 4°C. The supernatant was mixed with 1 ml of Ni-NTA agarose (Quiagen) pre-equilibrated with homogeneization buffer + 10 mM imidazole pH9 (buffer B). The mix was homogeneized for 2 hours at 4°C to allow the protein to bind to the Ni-NTA agarose beads. The beads were packed in a 1ml polypropylene column (Quiagen) and the resin was washed with 20 ml of the buffer B. The protein was eluted with 5 ml (5 X 1 ml) of buffer B + 250 mM imidazole pH9. Aliquots of the fractions were kept to follow the activity of the enzyme during the purification. To avoid any inhibition of the enzyme activity by high concentration of imidazole, the eluted fractions were dialysed against 4 liters of buffer containing Tris-HCl pH8, 0.1M, PMSF 100µM and ZnCl2 2µM overnight at 4°C. Thus, 1000µg proteins were recovered. The homogenised pellet was diluted at 3µgl/µl in a buffer containing 50 mM Tris-HCl pH8, 10% glycerol and 100µM PMSF, aliquoted and stored at -80°C. (Dilution D10 000).

### EXAMPLE 2: SINGLE STRAND SPECIFIC DNA DEGRADATION

The activity of the recombinant Cel I on degradation of single strand DNA was carried out as described previously (SUNG SC, LAKKOWSKI M Sr.(1962) J Biol Chem. 1962 Feb;237:506-11). Thirty micrograms of Dnase, Rnase and protease free Pea genomic DNA was incubated with 2 µg of protein extract in a buffer containing 50mM Tris-HCl (pH 7.6), 10mM MgCl2, 1mM DTT and 5% PEG-8000. To stop the reaction equal volume of 20mM LaCL3 in 0.2N HCl was added. The samples were centrifuged at 21000g for 40 min and the absorbance at 260nm of the supernatant was measured using spectrophotometer to determine the amount of DNA that had become acid-soluble.

### EXAMPLE 3: CLEAVAGE OF HETERODUPLEX DNA AND DETECTION OF A KNOWN POINT MUTATION IN A TEST GENE ON AGAROSE AND ACRYLAMIDE GELS BY THE ENDONUCLEASE PRODUCED IN TOBACCO

To test if the CEL I endonuclease produced in tobacco can recognize single point mutations, the activity of the recombinant CEL I preparation obtained by ammonium sulphate precipitation was tested on heteroduplex DNA from two clones that differ in a single point mutation: C-G to A-T transition (Bendhamane et al., Plant Cell, 11, 781-792, 1999). PCR was carried out on the two clones, using two oligonucleotides R21 and R22 (R21 5' GAC ATA TGG ACT ACA GAA GCT TGG G 3' SEQ ID N°8; R22 5' GTT CAC GGG TCA CAT CAT GCA TTC C 3' SEQ ID N°9). The PCR amplification and the reconstitution of heteroduplex DNA was carried out using the following program: denaturation for 2 min at 95°C followed by 7 cycles with 20sec at 94°C, Tm (55°C) +3°C to Tm -4°C for 15s, -1°C per cycle, gradient to 72°C at 0.5°C /sec and a an extension at 72°C for 1 min, then 44 cycles with 20sec at 94°C, Tm -5°C for 30°C, gradient to 72°C at 0.5°C/sec, and a extension at 72°C for 1 min, a final extension at 72°C for 5 min and a denaturation step at 94°C for 10 min followed by a ramp to 40°C for 20sec and -0.3°C per cycle.

The PCR products (a mix of wild type and mutant DNA, or just wild type or mutant DNA) were incubated with the CEL I preparation (stock solution at 1µg/µl diluted at 1/100, 1/500, or 1/1000) as follows: For example, 10 µl of the PCR product (500ng) was incubated with 2.5µl of the reaction buffer (Hepes 10mM, MgSO4 10mM, Triton X100 0.002%, KCl 10mM) and 2.5µl of the diluted CEL I preparation in a total volume of 25µl, for 30 minutes at 37°C. The reaction was stopped by 5µl of EDTA 500mM and the digestion products were analysed on a 3% agarose gel.

As shown on Figure 1, the detection of the point mutation in the test gene, Rx, on heteroduplex DNA is revealed by the appearance of two bands of about 200 bp and 400 bp at dilutions 1/100, 1/500 and 1/1000. These bands do not appear when no enzyme is added.

PCR products obtained using fluorescently labelled primers and digested in the same way as above were analysed on acrylamide gel on ABI377 sequencer (Figure 2).

As shown in figure 2, the two bands at 256 and 405 bp appear only when the Wt and Mut DNAs have been mixed together because of the formation of heteroduplexes between the two DNAs. This appears even more clearly when using different length waves for visualizing the gel. In particular, the band at 256 bp, labeled with the ROX fluorochrome (red), and not very clearly individualized on the black and white figure 2, is clearly present when the WT+Mut mix is used as PCR template, and absent in the other cases.

These results show that the CEL I protein produced *in planta* is able to recognize mismatches in DNA.

### EXAMPLE 4: DETECTION OF POINT MUTATIONS IN GENOMIC DNA FROM PEA BY THE RECOMBINANT CEL I

To test whether the recombinant CEL I purified from tobacco could be used to detect single point mutations in pea, different pea *rms* and *le* mutants, characterised previously by Catherine Rameau (MORUS et al Plant Physiol 2001, 126 :1205-1213. ; RAMEAU et al, Plant Physiol 2002, 115 :458-467), were used as a test; rms 1.11 contains G---->A at different positions in the sequence of the gene; rms 1-12 contains G---->A; rms 1-13 contains G---> A. All were mutated for G---->A on the same gene rms1 but at different positions.

To amplify the wild type and the mutant alleles of the *rms* loci (*rms1-13, rms1-10, rms1-12*) and le locus, fifty nanograms of pea genomic DNA was used as template. The primers used in this PCR amplification are summarised in Table 4.

**TABLE 4:**

| Pea mutant name | Name of the primer | Sequence 5' -> 3' |
|---|---|---|
| *rms 1-10* | 4m118 | 5'TTGGTTGGACTTCACTTTGAGC 3' SEQ ID N°10 |
| | 4m984 | 5' CACAACAATCAGCAATGACAGC 3' SEQ ID N°11 |
| *rms* 1-12 | 4-347 | 5' GTGATTGCTCCACCTCCGCCACC 3' SEQ ID N°12 |
| | 4-134 | 5'TACAGCGATTGATATAATATAAAATTATCC 3' SEQ ID N°13 |
| *rms 1-13* | 4-960 | 5' GTGTTTGTCCAGTAATAGTGTCAGCATA 3' SEQ ID N°3 |
| | 4-721 | 5' AGGAACCTGAGAAAAGACTCGCCAGC 3' SEQ ID N°4 |
| *le 1* | le 2462 | 5' TGATATTGTCGTGCAATATGATGAAAC 3' SEQ ID N°14 |
| | le 3082 | 5'ATACCTATTTAGCCCACTTGGACAC 3' SEQ ID N°15 |

The program of the PCR amplification was 94°C 1min, (94°C 15s, 55°C 15s, 74°C 1min, X35) 74°C 7 min, 8°C. The PCR products were analysed on agarose gel and digested, as disclosed in Example 2 above, with different dilutions of recombinant CEL I preparation obtained by ammonium sulphate precipitation. As shown on figure 3, the size of the PCR product for the rms 1-13 mutant and wild type mutant, is around 500 bp (481bp exactly). As a result of the detection of the mutation in heteroduplex DNA two bands of approximatively 200 and 300 bp were obtained. The two bands can be seen in an agarose gel even at a dilution of 1/1000 of the protein produced in tobacco. This result shows that the protein produced *in planta* is first, able to recognize a point mutation present in genomic DNA and second, very active since the digestion products can be seen even if the protein is diluted at 1/1000.

### EXAMPLE 5: EFFICIENCY OF MISMATCH CLEAVAGE AT DIFFERENT MISMATCHES BY THE RECOMBINANT CEL I

To test whether the recombinant CEL I prepared according to the invention cuts preferentially a type of mismatches, like the CEL I purified from celery, a series of mutants based on the Rx gene were created. For that purpose, we have designed different primers (called Rx-A, T, G or C) containing each, a different point mutation. Each of these primers allows to introduce at a given position of the Rx gene, one of the 4 bases (A, T , G or C). Heteroduplexes are created by mixing the amplification products obtained with the different primers.

The PCR mix (in total volume of 50µl) contained template DNA (50ng), dNTP (0.2mM), 5 µl PCR tampon Pfu (10X, Stratagene), primer Rx 21 (0.4µM), primer Rx-A or Rx-T or Rx-G or Rx-C (0.4µM), Pfu (5U, Stratagene, 2.5Unit/µl). The program used for PCR amplification was 94°C 1min, (94°C 15s, 55°C 15s, 74°C 2min, X35) 74°C 7 min, 8°C overnight. Labeled oligonucleotides with IRD700 and IRD800 fluorophore (MWS®) were used for the PCR to allow mismatch detection on LICOR4300 (LICOR ®).

The PCR products were analysed on agarose gel, and cloned in pGEM 3Zf. All the clones have been sequenced to make sure that the correct mutation has been inserted. The combinations of these constructs as template in PCR amplification were used to reconstitute all the types of mismatches.

Mismatched PCR product were incubated as disclosed in Example 2 above, with a recombinant CEL I preparation obtained from tobacco leaves by ammonium sulphate precipitation as disclosed in Example 1 above.

The products of the digestions were analyzed on acrylamide gel. A denaturating 6.5% acrylamide gel has been used, and electrophoretic conditions were: 1500V, 40W, 40mA, 45°C with a scan speed of 1.

The results are shown on figure 4 (Licor Gel)..

These results show that the CEL I preparations of the invention recognize all types of mismatches and particularly mismatches reported in the prior art as weakly recognized by CEL I purified directly from celery. Indeed, as shown in Table 2 above, CEL I preparation of the invention recognizes mismatches weakly recognized by CEL I preparation of the prior art, like T/T, G/A, A/G, G/T, T/G with very high specificity, and the mismatch preference of this enzyme is as follows: T/G ∼ A/G ∼ G/G ∼ G/T > T/T ∼ G/A ∼A/A ∼ C/C >T/C ∼ C/T ∼ A/C ∼ C/A.

This specific activity was also enhanced when the CEL I reaction buffer was supplemented with 5% PEG (data not shown).

### EXAMPLE 6: SENSITIVITY OF THE RECOMBINANT CEL I

To verify that the recombinant CEL I of the invention can be used for high throughput genotyping, it was tested whether it can recognize mutations in a pool of individuals.

Genomic DNA with a known SNP (C--> T), corresponding to the dwarf pea mutant *Le1*, and genomic DNA from wild type pea cultivar Torstag, have been used to amplify the *Le1* locus. These genomic DNA have been used as controls for homoduplex and heteroduplex formation.

To create heteroduplex DNA Genomic DNA derived from pea plant homozygote for the *le1* locus was diluted with genomic DNA derived from pea plant homozygote for the *Le1* locus in different proportions and 30ng was used for PCR amplification using the primers Le2462 labelled with TET fluorochrome (5'-TGATATTGTCGTGCAATATGATGAAAC-3' SEQ ID N°14) and Le3082 labelled with ROX (MWG®) fluorochrome (5'-ATACCTATTTAGCCCACTTGGACAC-3' SEQ ID N°15). The PCR reactions were carried out as follows: 94°C 1min,° (94°C 15s, 55°C 15s, 74°C 1 min ) X35, 74°C 7 min. Heteroduplex DNAs reconstituted from the PCR products were used as template in the mismatch detection assay as described above.

The results of this pooling experiment using the protein produced in tobacco are presented in Figure 5. As expected, our recombinant protein is able to recognize the SNP *Le1* anytime a heteroduplex has been formed between a wild type strand and a mutant DNA strand. As a result of the digestion, 2 bands at the expected size have been obtained (300 bp and 338 bp) and also a 3^{rd} band corresponding to the undigested homoduplex (638bp). The 338 bp band, which is labeled in red, appears clearly on the gel (but is less visible in black and white). Most interestingly, this mutation can be detected by the protein even when a pool of 24 different genomic DNAs has been used (addition of *Le1* and wild type with 23 unknown genomic DNAs).

To conclude, the protein produced *in planta* has a high sensitivity, allowing the identification of a known SNP within amplified DNA sequences coming from genomic DNA of at least 24 individuals.

### EXAMPLE 7: BIOINFORMATIC RESEARCH FOR DIFFERENT ENDONUCLEASES IN ARABIDOPSIS

An analysis of a family of genes from *Arabidopsis* coding for endonucleases has been done, using the protein profile PF02265 from the database PFAM. This profile HMM was used as a probe to target all the 27117 predicted proteins in *Arabidopsis* genome and the totality of the genome translated in the 6 different frameshifts to avoid the effect of the automatically structural annotation. In this analysis we identified 5 candidate genes At1g11190, At1g68290, At4g21590, At4g21585 and At4g21600.

### EXAMPLE 8 : CLONING AND EXPRESSION OF ARABIDOPSIS CANDIDATE GENES IN TOBACCO LEAVES

The cDNA of each candidate gene was PCR amplified and inserted between the 35S promoter and the transcriptional terminator of CaMV in the binary vector pBin61 to create pBIN35S-ENDO1, -ENDO2, -ENDO3, -ENDO4 and - ENDO5 that correspond to At1g11190, At1g68290, At4g21585, At4g21590 and At4g21600, respectively (Table 5). These constructs were transformed into Agrobacterium strain C58C1 carrying the virulence helper plasmid pCH32 (HAMILTON CM, et al. (1996) Proc Natl Acad Sci U S A. , 93(18):9975-9). pCH32 expresses VirG and VirE and was used to enhance T-DNA transfer. Agrobacterium cells were inoculated into 2 mL of L broth medium (SAMBROOK et al. 1989) supplemented with 50 µg/mL kanamycin and 5 µg/mL tetracyclin and grown at 28°C overnight. Cells were precipitated and resuspended to a final concentration of 0.5 OD₆₀₀ in a solution containing 10 mM MgCl₂, 10 mM MES, pH 5.6, and 150 µM acetosyringone. The cultures were incubated at room temperature for 2 hr before agroinfiltration into *Nicotiana benthamiana* leaves. Agroinfiltrated *Nicotiana benthamiana* plants were incubated for at least 24 hours at 24°C, 16 hours of light, 60 % of humidity. To test for the efficiency of agroinfiltration, plants were also agroinfiltrated with a construct expressing the green fluorescent protein (GFP). Before harvesting the leaves, the intensity of the expression of the GFP for each leaf was checked using an UV lamp. The plant leaves were harvested only if the GFP was expressed. The candidate proteins were extracted by ammonium sulfate precipitation as disclosed in Example 1.

**Table 5: Sequences of the primers used for the cloning of the candidate endonuclease with or without the Histidine tag**

| Candidates proteins | AGI genes | Primers |
|---|---|---|
| ENDO5 | **At4g21600** | Forward: AAGGATCCGAAAGCTCTGTGTTTCAGA **SEQ ID N0 :16** |
| | | Reverse:GGAGTTGTTACGTGGGTTCTCAAGGATC **SEQ ID N0 :17** |
| ENDO4 | At4g21585 | Forward:CTGGATCCCTGTTTTTAACTTTGGAAAG **SEQ ID N0 :18** |
| | | Reverse: GGATGTTCAAGTGATTCTCCTGGATC **SEQ ID N0 :19** |
| ENDO3 | At4g21590 | Forward:AAGGATCCATTCGACAAACTTTGTAAC **SEQ ID N0 :20** |
| | | Reverse:AGAGTGGTCTTGGGAATATTTATCTCAG **SEQ ID N0 :21** |
| ENDO2 | At1g68290 | Forward:ACGGATCCCATTTCAAAGAACTCTGA **SEQ ID N0 :22** |
| | | Reverse: GACCAATCATTATGCTGTAACTTCAG **SEQ ID N0 :23** |
| ENDO1 **SEQ ID N0 :2** | At1g11190 | Forward: CAGGATCCAAGTTTCAAACTTGAAG **SEQ ID N0 :24** |
| | | Reverse: CGGTATGTCGGGTTTGGTTCAAGTGG **SEQ ID N0 :25** |

### EXEMPLE 9: BIOCHEMICAL CHARACTERIZATION OF THE CANDIDATE PROTEINS.

As a simple test to see if the candidate protein is active, we incubate some supercoiled plasmid with different dilutions of protein extract (80% ammonium sulfate precipitate). As a result of the presence of an endonuclease in the protein extract, the supercoiled structure should be relaxed and some new DNA bands will appear when you run the incubation medium on an agarose gel. Using the different dilutions we can compare the different endonucleases and see which one is the most active. The control was always recombinant Cel I endonuclease prepared by ammonium sulfate precipitation as disclosed in Example 1 above.

To screen for candidate proteins able to cleave heteroduplex DNA at the mismatch site, we can also use a quick characterisation system based on three consecutive steps.

In the first step the candidate proteins were tested for their ability to degrade single strand DNA. This condition was tested because the DNA at the mismatch site in heteroduplex DNA is single stranded. Thus, an endonuclease that is not able to digest single strand DNA is predicted not able to cleave DNA at the mismatch site. Second, the candidate protein should cleave a test heteroduplex DNA at known and well characterised mismatch site. Third, proteins that pass test 1 and 2 were evaluated for their efficiency to cleave heteroduplex DNA fragments carrying all the types of mismatches. This was carried out using DNA toolbox which consists of a set of well characterised plasmid constructs that contain at specific position of the insert, each of the four possible nucleotides.

### Ability to degrade a single strand DNA

The activity of the candidate proteins on degradation of single strand DNA was carried out as described in Example 2. In this analysis all the five candidate proteins showed nuclease activity and were classified from the most active to the less active as follows: ENDO1, ENDO5, END02, ENDO3 and ENDO4.

### Cleavage of heteroduplex DNA at C-A/T-G mismatch site

To test if the candidate proteins produced in tobacco can recognize single point mutations, we tested the activity of the protein extracts on heteroduplex DNA created as disclosed in Example 3. The PCR products containing each or both of the alleles were incubated with 1/1000 dilution of the protein extracts derived from leaves agroinfiltrated either with the candidate endonuclease gene or with the GFP as control, as follows: 500ng of the PCR products were incubated with tobacco protein extract in 25µl final volume containing 50mM Tris-HCl (pH 7.6), 10mM MgCl2, 1mM DTT and 5% PEG-8000 for 30 minutes at 37°C. The reactions were stopped with EDTA at the final concentration of 80mM and analysed on a 3% agarose gel

If the two oligonucleotides were fluorescently labelled the digestion products were analysed on acrylamide gel, on a 377Abi DNA sequencer. In this experiment, we predicted that if the protein extract contained a mismatch specific endonuclease, the heteroduplex DNA would be cleaved at the mismatch site, thereby releasing two bands of 256 bp and 405 bp.

The results are shown on Figure 6 (note that the 256 bp band, labelled in red, is clearly visible when the enzyme is in the presence of heteroduplex, and absent otherwise; this band is less distinguishable in black and white).

From this biochemical analysis, we concluded that the five endonucleases exhibit mismatch specific cleavage activities. Moreover, the three enzymes, ENDO1, ENDO5, ENDO2, which have higher single strand DNA specific-nuclease activity, cleave also with higher efficiency heteroduplex DNA at the C-A/T-G mismatch site. Furthermore, among these three endonucleases, ENDO1 and ENDO5 were the most active ones. Thus, these two nucleases were selected for further accurate characterisation.

### Efficiency of mismatch cleavage by the candidate endonuclease at different mismatches

The main goal of this task is to identify an endonuclease that cleaves mismatches that CEL-I does not recognise efficiently. This was carried out using DNA toolbox which consists of a set of well characterised plasmids constructs that contain at specific position of the insert each of the four possible nucleotides. The combination of these constructs as template in PCR amplification was used to reconstitute all the types of mismatches.

Mismatched PCR product were incubated as above (see example 5) with the candidate endonuclease and analysed on the LICOR sequencing machine.

The results are shown on Figure 7.

In this analysis, ENDO5 like CEL-I recognises weakly the T/T type of mismatches. In contrast, ENDO1 recognises nearly all the type of mismatches with high efficiency. From this analysis, we can conclude that ENDO1 is able to recognise mismatches undetected by CEL-I.

### EXAMPLE 10: SENSITIVITY OF THE ENDO1 TO DETECT A MUTANT ALLELE IN A DNA POOL

The sensitivity of ENDO1 has been evaluated as described in Example 3, with dilutions of 2, 4, 6, 8, 10, 15, 20, 25, 30, 35, 40, 50 or 60 fold with DNA carrying the wild-type allele.

The results of this pooling experiment are presented in Figure 8, which shows the detection of one allele from a homozygote mutant among 60 alleles from a homozygote wild-type. PCR from the two organisms have been done and amplicons have been quantified on agarose gel, and mixed together following a decreasing ratio from 1:1 to 1:60 of the PCR amplicons. ENDO1 activity has then been assayed on those different ratios of mutant:wild-type.

In identical experiments, CEL-I purified from celery can detect one allele in a maximum of 16, with low sensitivity, and a correct sensitivity is obtained only when the dilution is inferior or equal to 8-fold.

In conclusion, endonucleases according to the invention and particularly ENDO1 have a much less background noise than Cel I, can be used at very high dilution compared to Cel I and have better specificity and activity than Cel I endonuclease.

### Detection of point mutation on acrylamide gel.

A series of mutants based on Rx gene were created. We have designed different plasmids containing each one type of mismatch in order to show the specificity of ENDO1. The PCR mix contains oligonucleotides specific to the plasmids. Labeled oligonucleotides with ROX and FAM fluorophore (MWG®) were used for the PCR to allow mismatch detection on ABI377 MWG®.

As shown in figure 9, ENDO1 does not cut when only homoduplexes are present, for example line 1 or line 5, and the only bands that we can see on the gel are homoduplexes at the top of the gel (around 600 bp).

Anytime we have a mismatch in our samples (resulting from the formation of heteroduplex between two strands of DNA coming from 2 different plasmids, as for example on lines 2, 3, 4 or 6, 7 and 9) the ENDO 1 recognizes and cuts at the site of the mismatch, resulting in the appearance of two bands corresponding to the two products, each labelled with one fluorophore. For the ENDO 1 digestion, a decrease in the background can be seen and some mismatches can be detected only with ENDO 1 (line 9 for example)

### Detection of a known point mutation on acrylamide gel of ENDO1 in two different dilutions D1000 and D5000

Two plasmids containing a wild form and a mutated form of Rx gene were used. They differ only by one known point mutation. The PCR mix contains oligonucleotides specific to the plasmids. Labelled oligonucleotides with ROX and FAM fluorophores (MWG®) were used for the PCR to allow mismatch detection on ABI377 MWG®.

As shown on figure 10, anytime a heteroduplex is present in our samples (M5+M12), ENDO1 is able to recognize and cut at the site of the mismatch, resulting in the appearance of two bands labelled with a fluorophore. When only M5 or M12 homoduplexes are present, ENDO 1 does not cut and the only bands that we can see on the gel are homoduplexes at the top of the gel (around 600 bp). An improvement of the background can be seen when we have used the D 5000 for the ENDO1. Therefore, Endol can be used a very high dilution compared to any endonucleases known in the art.

## Claims

1. A method for producing a recombinant mismatch-specific endonuclease wherein said method comprises:
- expressing said recombinant endonuclease in cells of a whole host plant or of an organ detached therefrom, transiently transformed by agroinfiltration with an *Agrobacterium* strain containing an expression vector comprising a polynucleotide encoding said endonuclease;
- isolating said recombinant endonuclease from said host plant cells.

2. A method of claim 1, wherein said agroinfiltration is performed in a leaf of said host-plant.

3. A method of any of claims 1 or 2, wherein said host plant belongs to the genus *Nicotiana.*

4. A method of any of claims 1 to 3, wherein said endonuclease is isolated from the agroinfiltrated plant organ by a process comprising the following steps:
- extracting the cell content from the agroinfiltrated organ expressing said endonuclease;
- adding ammonium sulfate at a final concentration of at least 30 % to said extract, and separating the protein precipitate from the supernatant;
- adding ammonium sulfate at a final concentration of at least 80 % to said supernatant, and recovering the protein precipitate containing the endonuclease.

5. The method of claim 4, wherein the ammonium sulphate is added at a final concentration of 30% in the first precipitation step, and at a final concentration of 80% in the second precipitation step.

6. A preparation of recombinant mismatch-specific endonuclease obtainable by the method of any of claims 1 to 5, wherein said endonuclease is selected among CEL I from *Apium graveolens* and BFN1 from *Arabidopsis thaliana* of SEQ ID NO:2.

7. A preparation of recombinant CEL I endonuclease of claim 6, wherein said recombinant CEL I endonuclease has the following mismatch preference: T/T ∼ T/G ∼ A/G ∼ G/G ∼ G/A ∼ G/T ≥ A/A ∼ C/C ≥T/C ∼ C/T > A/C ∼ C/A, and is able to recognize a mutant allele in the presence of a 23-fold-excess of the wild type allele.

8. A preparation of recombinant BFN1 endonuclease of claim 6, wherein said recombinant BFN1 endonuclease has the following mismatch preference: G/G ∼ G/A ∼ A/G ∼ G/T ∼ T/G > T/T ∼ A/A ∼ C/C ∼ T/C > C/T ∼ A/C ∼ C/A, and is able to recognize a mutant allele in the presence of a 59-fold excess of the wild type allele.

9. Use of a preparation of recombinant endonuclease of any of claims 6 to 8, for detecting in a DNA duplex, a mismatch resulting from a base substitution, or from insertion or deletion of one or more nucleotides in one strand of said duplex.

10. Use of a recombinant endonuclease preparation according to anyone of claims 6 to 8, in a Targeting-Induced Local Lesions IN Genomes (TILLING) mismatch cleavage protocol.

11. Use of a recombinant endonuclease preparation according to anyone of claims 6 to 8, for the identification of DNA polymorphisms in natural populations, by Ecotilling.

12. Use of a recombinant endonuclease preparation according to anyone of claims 6 to 8, as a mismatch detecting reagent.

13. Use of a recombinant endonuclease preparation according to anyone of claims 6 to 8, in a method of mismatch screening.

14. Use of a recombinant endonuclease preparation according to anyone of claims 6 to 8, for simultaneously screening one or more mutations in a target gene in a population of any organism or cell-line derived therefrom, by performing the steps of:
a) amplifying said target gene or part thereof for each individual of said population,
b) ordering said amplifications product in a 2- or 3-dimensional matrix, comprising lines, rows (2-D matrix) and columns (3-D matrix),
c) pooling said amplification products such as to obtain different pools, each pool representing a row, a line or a column of said matrix,
d) adding to each pool a reference amplification product obtained from a non-mutated gene, and incubating such pools in conditions permitting formation of heteroduplexes, and
e) incubating each pool with said endonuclease preparation, and
f) detecting the presence of heteroduplexes in said incubated pools.

15. Use of a recombinant endonuclease preparation according to anyone of claims 6 to 8, for simultaneously screening one or more mutations in a target gene in a population of any organism or cell-line derived therefrom, by performing the steps of:
a) ordering each individuals of said population, in a 2- or 3-dimensional matrix, comprising lines, rows (2-D matrix) and columns (3-D matrix),
b) pooling each row, line and column in order to obtain different pools, each pool thus representing a row, a line or a column of said matrix,
c) adding to each pool a reference gene product obtained from a non-mutated gene,
d) amplifying said target gene or part thereof in each pool in order to get pools of amplified products,
e) incubating said pools of amplified products in conditions permitting formation of heteroduplexes,
f) incubating said pools of amplified products with said endonuclease preparation, and
g) detecting the presence of heteroduplexes in said incubated pools.

## Patentansprüche

1. Verfahren zur Herstellung einer rekombinanten mismatch-spezifischen Endonuklease, wobei das Verfahren umfasst:
- Exprimieren der rekombinanten Endonuklease in Zellen einer ganzen Gastpflanze oder eines Organs, das davon abgetrennt ist, transient transformiert durch Agroinfiltration mit einem *Agrobacterium* Strang, das einen exprimierten Vektor enthält, der ein Polynukleotid umfasst, das die Endonuklease N codiert;
- Isolierung der rekombinanten Endonuklease von den Gastpflanzenzellen.

2. Verfahren nach Anspruch 1, wobei die Agroinfiltration in einem Blatt der Gastzelle durchgeführt wird.

3. Verfahren nach einem der Ansprüche 1 oder 2, wobei die Gastpflanze zu dem Genus *Nicotiana* gehört.

4. Verfahren nach einem der Ansprüche 1 bis 3, wobei die Endonuklease aus dem agroinfiltrierten Pflanzenorgan durch ein Verfahren isoliert wird, das die folgenden Schritte umfasst:
- Extraktion des Zellgehaltes aus dem agroinfiltrierten Organ, das die Endonuklease exprimiert;
- Zugabe von Ammoniumsulfat auf eine Endkonzentration von mindestens 30% zu dem Extrakt und Separierung des Proteinniederschlags aus dem Überstand;
- Zugabe von Ammoniumsulfat auf eine Endkonzentration von mindestens 80% zu dem Überstand und Gewinnung des Proteinniederschlags, der die Endonuklease enthält.

5. Verfahren nach Anspruch 4, wobei Ammoniumsulfat auf eine Endkonzentration von 30% in dem ersten Fällungsschritt zugefügt wird, und auf eine Endkonzentration von 80% in dem zweiten Fällungsschritt.

6. Präparation einer rekombinanten mismatch-spezifischen Endonuklease, die durch ein Verfahren gemäß einem der Ansprüche 1 bis 5 erhalten wird, wobei die Endonuklease ausgewählt ist aus CEL I aus *Apium graveolens* und BFN1 aus *Arabidopsis thaliana* aus SEQ ID NO: 2.

7. Präparation einer rekombinanten CEL I Endonuklease nach Anspruch 6, wobei die rekombinante CEL I Endonuklease die folgende Mismatch-Präferenz aufweist: T/T ∼ T/G ∼ A/G ∼ G/G ∼ G/A ∼ G/T ≥ A/A ∼ C/C ≥ T/C ∼ C/T > A/C ∼ C/A, und geeignet ist, ein mutantes Allel in Anwesenheit eines 23-fachen Überschusses des Wildtyp Allels zu erkennen.

8. Präparation einer rekombinanten BFN1 Endonuklease nach Anspruch 6, wobei die rekombinante BFN1 Endonuklease die folgende Mismatch-Präferenz aufweist: G/G ∼ G/A ∼ A/G ∼ G/T ∼ T/G > T/T ∼ A/A ∼ C/C ∼ T/C > C/T ∼ A/C ∼ C/A, und geeignet ist, ein mutantes Allel in Anwesenheit eines 59-fachen Überschusses des Wildtyp Allels zu erkennen.

9. Verwendung einer Präparation der rekombinanten Endonuklease gemäß einem der Ansprüche 6 bis 8 zur Feststellung eines Mismatches in einer DNA Doppelhelix, der aus einer Basensubstitution oder einer Insertion oder Löschung aus einem oder mehreren Nukleotiden in einem Strang der Doppelhelix resultiert.

10. Verwendung einer rekombinanten Endonukleasepräparation gemäß einem der Ansprüche 6 bis 8 in einem Targeting-induzierten Lokalläsion-IN-Genom (TILLING) -Mismatch Spaltungsprotokoll.

11. Verwendung einer rekombinanten Endonuklease Präparation gemäß einem der Ansprüche 6 bis 8 zur Identifikation von DNA Polymorphismen in natürlichen Populationen durch Ecotilling.

12. Verwendung einer rekombinanten Endonukleasepräparation gemäß einem der Ansprüche 6 bis 8 als ein Mismatchdetektionsreagenz.

13. Verwendung einer rekombinanten Endonukleasepräparation gemäß einem der Ansprüche 6 bis 8 in einem Verfahren zum Mismatchscreening.

14. Verwendung einer rekombinanten Endonukleasepräparation gemäß einem der Ansprüche 6 bis 8 zum gleichzeitigen Screenen von einer oder mehreren Mutationen in einem Targetgen in einer Population von irgendeinem Organismus oder einer daraus abgeleiteten Zelllinie, bei dem die folgenden Schritte durchgeführt werden:
a) Verstärkung des Targetgens oder eines Teils davon für jede individuelle Population,
b) Anordnung dieser Verstärkungsprodukte in einer 2- oder 3-dimensionalen Matrix umfassend Linien, Reihen (2D Matrix) und Säulen (3D Matrix),
c) Poolbindung der Verstärkungsprodukte, so dass die verschiedenen Pools erhalten werden, wobei jeder Pool, eine Reihe, eine Linie oder eine Säule der Matrix darstellt,
d) Zugabe eines Referenzverstärkungsproduktes zu jedem Pool, das aus einem nichtmutierten Gen erhalten wird und Inkubation dieser Pools zu Bedingungen, die die Bildung einer Heterodoppelhelix erlauben, und
e) Inkubation von jedem Pool mit der Endonukleasepräparation, und
f) Feststellung der Anwesenheit der Hetereodoppelhelices in den inkubierten Pools.

15. Verwendung einer rekombinanten Endonukleasepräparation gemäß einem der Ansprüche 6 bis 8 zum gleichzeitigen Screenen von einer oder mehreren Mutationen in einem Targetgen in einer Population von irgendeinem Mechanismus oder einer davon abgeleiteten Zelllinie, bei dem die folgenden Schritte durchgeführt werden:
a) Anordnung jeder Individuen der Population in einer 2- oder 3-dimensionalen Matrix umfassend Linien, Reihen (2D Matrix) und Säulen (3D Matrix),
b) Poolbildung jeder Reihe, Linie oder Säule, um verschiedene Pools zu erhalten, wobei jeder Pool somit eine Reihe, eine Linie oder eine Säule der Matrix darstellt,
c) Zugabe eines Referenzengenproduktes zu jedem Pool, das aus einem nichtmutierten Gen erhalten wurde,
d) Verstärkung des Targetgens oder eines Teils davon in jedem Pool, um Pools von verstärkten Produkten zu erhalten,
e) Inkubation der Pools der verstärkten Produkte zu Bedingungen, die die Bildung von Heterodoppelhelices erlaubt,
f) Inkubation der Pools der verstärkten Produkte mit der Endonukleasepräparation,
g) Feststellung der Anwesenheit von Heterodoppelhelices in den inkubierten Pools.

## Revendications

1. Procédé de production d'une endonucléase recombinante spécifique des mésappariements, ledit procédé comprenant :
- l'expression de ladite endonucléase recombinante dans des cellules d'un hôte végétal entier ou d'un organe séparé de ce dernier, transformé transitoirement par agroinfiltration avec une souche *d'Agrobacterium* contenant un vecteur d'expression comprenant un polynucléotide codant ladite endonucléase ;
- l'isolement de ladite endonucléase recombinante desdites cellules végétales hôtes.

2. Procédé selon la revendication 1, dans lequel ladite agroinfiltration est effectuée sur une feuille dudit hôte végétal.

3. Procédé selon l'une quelconque des revendications 1 ou 2, dans lequel ledit hôte végétal appartient au genre *Nicotiana.*

4. Procédé selon l'une quelconque des revendications 1 à 3, dans lequel ladite endonucléase est isolée de l'organe végétal agroinfiltré par un procédé comprenant les étapes suivantes :
- l'extraction du contenu des cellules issues de l'organe agroinfiltré exprimant ladite endonucléase ;
- l'ajout de sulfate d'ammonium à une concentration finale d'au moins 30 % audit extrait, et la séparation entre le précipité protéique et le surnageant ;
- l'ajout de sulfate d'ammonium à une concentration finale d'au moins 80 % audit surnageant, et la récupération du précipité protéique contenant l'endonucléase.

5. Procédé selon la revendication 4, dans lequel le sulfate d'ammonium est ajouté à une concentration finale de 30 % dans la première étape de précipitation et à une concentration finale de 80 % dans la seconde étape de précipitation.

6. Préparation d'endonucléase recombinante spécifique des mésappariements pouvant être obtenue par le procédé selon l'une quelconque des revendications 1 à 5, dans laquelle ladite endonucléase est choisie parmi CEL I provenant *d'Apium graveolens* et BFN1 provenant *d'Arabidopsis thaliana* de SEQ ID NO : 2.

7. Préparation d'une endonucléase CEL I recombinante selon la revendication 6, dans laquelle la préférence de ladite endonucléase CEL I recombinante en ce qui concerne les mésappariements est la suivante : T/T ∼ T/G ∼ A/G ∼ G/G ∼ G/A ∼ G/T ≥ A/A ∼ C/C ≥ T/C ∼ C/T > A/C ∼ C/A, et est capable de reconnaître un allèle mutant en présence de 23 fois plus d'allèle de type sauvage.

8. Préparation d'une endonucléase BFN1 recombinante selon la revendication 6, dans laquelle la préférence de ladite endonucléase BFN1 recombinante en ce qui concerne les mésappariements est la suivante : G/G ∼ G/A ∼ A/G ∼ G/T ∼ T/G > T/T ∼ A/A ∼ C/C ∼ T/C > C/T ∼ A/C ∼ C/A et est capable de reconnaître un allèle mutant en présence de 59 fois plus d'allèle de type sauvage.

9. Utilisation d'une préparation d'endonucléase recombinante selon l'une quelconque des revendications 6 à 8, pour détecter dans un ADN double brin, un mésappariement résultant d'une substitution de base ou de l'insertion ou de la délétion d'un ou de plusieurs nucléotides dans un brin dudit ADN double brin.

10. Utilisation d'une préparation d'endonucléase recombinante selon l'une quelconque des revendications 6 à 8, dans un protocole de clivage de mésappariement par ciblage de lésions induites localement dans les génomes (TILLING).

11. Utilisation d'une préparation d'endonucléase recombinante selon l'une quelconque des revendications 6 à 8, pour l'identification des polymorphismes de l'ADN de populations naturelles, par Ecotilling.

12. Utilisation d'une préparation d'endonucléase recombinante selon l'une quelconque des revendications 6 à 8, en tant que réactif de détection des mésappariements.

13. Utilisation d'une préparation d'endonucléase recombinante selon l'une quelconque des revendications 6 à 8, dans un procédé de criblage de mésappariements.

14. Utilisation d'une préparation d'endonucléase recombinante selon l'une quelconque des revendications 6 à 8, pour cribler simultanément une ou plusieurs mutations dans un gène cible d'une population de n'importe quel organisme ou d'une lignée cellulaire en dérivant, en mettant en oeuvre les étapes suivantes :
a) l'amplification dudit gène cible ou de l'une de ses parties pour chaque individu de ladite population,
b) le classement desdits produits d'amplification dans une matrice bi- ou tri-dimensionnelle, comprenant des lignes, des rangées (matrice 2-D) et des colonnes (matrice 3-D),
c) le regroupement desdits produits d'amplification de manière à obtenir différents pools, chaque pool représentant une rangée, une ligne ou une colonne de ladite matrice,
d) l'ajout à chaque pool d'un produit d'amplification de référence obtenu à partir d'un gène non muté et l'incubation de ces pools dans des conditions permettant la formation d'hétéroduplex, et
e) l'incubation de chaque pool avec ladite préparation d'endonucléase, et
f) la détection de la présence des hétéroduplex dans lesdits pools incubés.

15. Utilisation d'une préparation d'endonucléase recombinante selon l'une quelconque des revendications 6 à 8, pour cribler simultanément une ou plusieurs mutations dans un gène cible d'une population de n'importe quel organisme ou d'une lignée cellulaire en dérivant, en mettant en oeuvre les étapes suivantes :
a) le classement de chaque individu de ladite population dans une matrice bi- ou tri-dimensionnelle, comprenant des lignes, des rangées (matrice 2-D) et des colonnes (matrice 3-D),
b) le regroupement de chaque rangée, ligne et colonne afin d'obtenir différents pools, chaque pool représentant ainsi une rangée, une ligne ou une colonne de ladite matrice,
c) l'ajout à chaque pool d'un produit génétique de référence obtenu à partir d'un gène non muté,
d) l'amplification dudit gène cible ou de l'une de ses parties dans chaque pool afin d'obtenir des pools de produits amplifiés,
e) l'incubation desdits pools de produits amplifiés dans des conditions permettant la formation d'hétéroduplex,
f) l'incubation desdits pools de produits amplifiés avec ladite préparation d'endonucléase, et
g) la détection de la présence des hétéroduplex dans lesdits pools incubés.


### SEQUENCE LISTING

<110> GENOPLANTE-VALOR
   INSTITUT NATIONAL DE LA RECHERCHE AGRONOMIQUE
   BENDAHMANE, Abdelhafid
   STURBOIS, Bénédicte
   TRIQUES, Karine
   CABOCHE, Michel
<120> METHOD FOR PRODUCING HIGHLY SENSITIVE ENDONUCLEASES, NOVEL PREPARATIONS OF ENDONUCLEASES AND USES THEREOF.
<130> MJP/bv1516-19
<150> PCT/EP2004/009159
   <151> 2004-07-30
<150> PCT/EP2004/009166
   <151> 2004-07-30
<160> 25
<170> PatentIn version 3.3
<210> 1
   <211> 2640
   <212> DNA
   <213> Arabidopsis thaliana
<220>
   <221> misc_feature
   <223> ENDO 1 Gene At1g11190
<400> 1
   aaattcgatg aggttgttat agacaagaga agacattttt atacaaaaga gtttatcatt     60
   atataagttt caaactttga agatatggca tcggctttta gatcatccac gaggttgatt    120
   cttgtattag gtatactgat tttgtgttcg gtttcttctg tccgaagctg gagcaaagaa    180
   ggtcatattc ttacttgtag aattgctcag gtaattaagt taatgatcta ttgtttgaag    240
   caactatttt ggttattctt gtcttatata tgtattagtg agatatacct acaaattttt    300
   aattaggatt gacttttaaa ttgctatacg ttaccatgcc taacatctca tgtagatgat    360
   catgaataca aacatgtcta atggcatatc aaattccaag tttttttggt agagatctga   420
   gtcatttgac cgttataaga ttcataacaa aagttcgtat gtgtgtgttt ttgtggtgtg    480
   accagaatct tttagaagcc ggaccagcac atgtagtaga gaatctgtta ccggattacg    540
   tgaaaggaga tttatcagca ttgtgtgtgt ggcctgacca gatccgacat tggtacaagt    600
   atcgttggac cagccatctc cattacatcg acactcccga ccaagcctgc tcttacgaat    660
   actctagtaa gtcacaaccg agacattttc agataacctt aatccgtttt ctaattatct    720
   tgaaccggag ttaaccaaaa aatcaattac aaataccaaa ccggattaaa aacaggggat    780
   tgtcatgatc aacatggatt gaaggatatg tgtgtggatg gagcaatcca gaatttcacg    840
   tctcagcttc agcattacgg tgaaggaaca tctgatcgta gatgtatgtc atcattttca    900
   tttatttcat ataatgatga tatccaaagt gtaactgcgt attttgtatt ttgatgcata    960
   acttaagttt ttaaaattat aatatatcct tgttcaatca catagataac atgaccgaag   1020
   cccttttgtt cttgtctcat ttcatgggag atattcatca ggtttattac tcatcatcga 1080
   ttcatttcac acctccacac atatagctct atttccatgt taaatattta attaacatgg 1140
   tttttttttt tttccttaaa aagccgatgc atgtgggatt cacaagtgat gaaggaggaa 1200
   acacgataga tttacgttgg tacaaacaca aatccaatct acatcatgta agcttcttct 1260
   tttgtctctt tcaactttaa atttcatcat gaaaacaaaa aaaaaattaa cgaaggaaac 1320
   aaaatatgta ggtatgggat agagagatca ttctcacggc tctaaaagaa aactacgaca 1380
   agaacttgga tcttctccaa gaggatcttg agaagaacat caccaatgta atagacacta 1440
   atttattcat attttactat aattttaaga atctttataa tggttatcat atattaggga 1500
   ttatggcacg acgatctatc ttcgtggaca gaatgcaacg atcttatcgc ttgtccacac 1560
   aagtaagttt taaattactt ggtttaagat tggcttgacg ctcgtttgaa gctagctaca 1620
   aattttgata ctttttctgg tccaaaaatc ttacaaagat actgaaaata aaataatagg 1680
   ttttaaactt ttaatttatt tggagttgga taggattaag tttcactaac ttccaattca 1740
   aagtcaatta atagtagttt accatgatta gtgggttgac taatgtacca tatatattac 1800
   cttatatcac atcttatttc cgatgtgaga tttcttatga aacataatta gactcgaacc 1860
   ttttgtgttt cgatatatgt agtgtattca tgatcagaat cttattaagt ttacaactga 1920
   aaactaaaat attaacatca taattataga ttcttaagta ggttttgttt gggtggagaa 1980
   aatatccaat ttcgaataac attatataaa atattgaact aattttaatt gtatacgcag 2040
   gtatgcttca gagagtataa agttagcttg taaatgggga tacaaaggcg tcaagtctgg 2100
   tgaaacgtta tcaggtacgt tgtttgcttc ttctttttct cgtacgctaa caaaaatatt 2160
   taaaaataaa cccgaccaaa tgaagtttaa ttaatcggat taatgatttt taatagtcac 2220
   tacttttttt gtgtgggata tatgactgtc taatatataa ttttataaga aagctaaagg 2280
   atttgtttaa taatttccga taaataattt tgcagaagaa tatttcaata caaggttgcc 2340
   aatagtgatg aagagaatag ttcagggagg agttagacta gccatgatac taaaccgggt 2400
   ttttagtgac gatcatgcta ttgctggtgt tgctgccact tgaaccaaac ccgacatacc 2460
   ggggcatcaa agcatttgat taagagatta tttgatacat tcacaaaatt aattaaggct 2520
   gatcagacat tcttttcttt tagtagcttt atctatgtga cagctaatgc ctgtggactg 2580
   ctttgtttag aagtgtttag cattagatca tatgctaatt caatgttatt aattcatcgt 2640
<210> 2
   <211> 305
   <212> PRT
   <213> Arabidopsis thaliana
<220>
   <221> misc_feature
   <223> Protein ENDO 1 At1g11190
<400> 2
   Met Ala Ser Ala Phe Arg Ser Ser Thr Arg Leu Ile Leu Val Leu Gly
   1               5                   10                  15
   Ile Leu Ile Leu Cys Ser Val Ser Ser Val Arg Ser Trp Ser Lys Glu
               20                  25                  30
   Gly His Ile Leu Thr Cys Arg Ile Ala Gln Asn Leu Leu Glu Ala Gly
           35                  40                  45
   Pro Ala His Val Val Glu Asn Leu Leu Pro Asp Tyr Val Lys Gly Asp
       50                  55                  60
   Leu Ser Ala Leu Cys Val Trp Pro Asp Gln Ile Arg His Trp Tyr Lys
   65                  70                  75                  80
   Tyr Arg Trp Thr Ser His Leu His Tyr Ile Asp Thr Pro Asp Gln Ala
                   85                  90                  95
   Cys Ser Tyr Glu Tyr Ser Arg Asp Cys His Asp Gln His Gly Leu Lys
               100                 105                 110
   Asp Met Cys Val Asp Gly Ala Ile Gln Asn Phe Thr Ser Gln Leu Gln
           115                 120                 125
   His Tyr Gly Glu Gly Thr Ser Asp Arg Arg Tyr Asn Met Thr Glu Ala
       130                 135                 140
   Leu Leu Phe Leu Ser His Phe Met Gly Asp Ile His Gln Pro Met His
   145                 150                 155                 160
   Val Gly Phe Thr Ser Asp Glu Gly Gly Asn Thr Ile Asp Leu Arg Trp
                   165                 170                 175
   Tyr Lys His Lys Ser Asn Leu His His Val Trp Asp Arg Glu Ile Ile
               180                 185                 190
   Leu Thr Ala Leu Lys Glu Asn Tyr Asp Lys Asn Leu Asp Leu Leu Gln
           195                 200                 205
   Glu Asp Leu Glu Lys Asn Ile Thr Asn Gly Leu Trp His Asp Asp Leu
       210                 215                 220
   Ser Ser Trp Thr Glu Cys Asn Asp Leu Ile Ala Cys Pro His Lys Tyr
   225                 230                 235                 240
   Ala Ser Glu Ser Ile Lys Leu Ala Cys Lys Trp Gly Tyr Lys Gly Val
                   245                 250                 255
   Lys Ser Gly Glu Thr Leu Ser Glu Glu Tyr Phe Asn Thr Arg Leu Pro
               260                 265                 270
   Ile Val Met Lys Arg Ile Val Gln Gly Gly Val Arg Leu Ala Met Ile
   275 280 285
   Leu Asn Arg Val Phe Ser Asp Asp His Ala Ile Ala Gly Val Ala Ala
   290 295 300
   Thr
   305
<210> 3
   <211> 28
   <212> DNA
   <213> Artificial sequence
<220>
   <223> Primer 4-960
<400> 3
   gtgtttgtcc agtaatagtg tcagcata 28
<210> 4
   <211> 26
   <212> DNA
   <213> Artificial sequence
<220>
   <223> Primer 4-721
<400> 4
   aggaacctga gaaaagactc gccagc 26
<210> 5
   <211> 40
   <212> DNA
   <213> Artificial sequence
<220>
   <223> CEL N Terminal
<400> 5
   tatcgttcta gagggaatga cgcgattata ttctgtgttc 40
<210> 6
   <211> 27
   <212> DNA
   <213> Artificial sequence
<220>
   <223> CEL C Terminal
<400> 6
   tatctgaatt catgccaaag aatgatc 27
<210> 7
   <211> 50
   <212> DNA
   <213> Artificial sequence
<220>
   <223> CEL C terminal 8 His
<400> 7
   aattcaatgg tgatggtggt gatggtgatg tgccaaagaa tgatctgcgg 50
<210> 8
   <211> 25
   <212> DNA
   <213> Artificial sequence
<220>
   <223> Primer (R21)
<400> 8
   gacatatgga ctacagaagc ttggg 25
<210> 9
   <211> 25
   <212> DNA
   <213> Artificial sequence
<220>
   <223> Primer (R22)
<400> 9
   gttcacgggt cacatcatgc attcc 25
<210> 10
   <211> 22
   <212> DNA
   <213> Artificial sequence
<220>
   <223> Primer 4m118
<400> 10
   ttggttggac ttcactttga gc 22
<210> 11
   <211> 22
   <212> DNA
   <213> Artificial sequence
<220>
   <223> Primer 4m984
<400> 11
   cacaacaatc agcaatgaca gc 22
<210> 12
   <211> 23
   <212> DNA
   <213> Artificial sequence
<220>
   <223> Primer 4-347
<400> 12
   gtgattgctc cacctccgcc acc 23
<210> 13
   <211> 30
   <212> DNA
   <213> Artificial sequence
<220>
   <223> Primer 4-134
<400> 13
   tacagcgatt gatataatat aaaattatcc 30
<210> 14
   <211> 27
   <212> DNA
   <213> Artificial sequence
<220>
   <223> Primer le 2462
<400> 14
   tgatattgtc gtgcaatatg atgaaac 27
<210> 15
   <211> 25
   <212> DNA
   <213> Artificial sequence
<220>
   <223> Primer le 3082
<400> 15
   atacctattt agcccacttg gacac 25
<210> 16
   <211> 27
   <212> DNA
   <213> Artificial sequence
<220>
   <223> Forward Primer for ENDO5
<400> 16
   aaggatccga aagctctgtg tttcaga 27
<210> 17
   <211> 28
   <212> DNA
   <213> Artificial sequence
<220>
   <223> Reverse Primer for ENDO5
<400> 17
   ggagttgtta cgtgggttct caaggatc 28
<210> 18
   <211> 28
   <212> DNA
   <213> Artificial sequence
<220>
   <223> Forward Primer for ENDO4
<400> 18
   ctggatccct gtttttaact ttggaaag 28
<210> 19
   <211> 26
   <212> DNA
   <213> Artificial sequence
<220>
   <223> Reverse Primer for ENDO4
<400> 19
   ggatgttcaa gtgattctcc tggatc 26
<210> 20
   <211> 27
   <212> DNA
   <213> Artificial sequence
<220>
   <223> Forward Primer for ENDO3
<400> 20
   aaggatccat tcgacaaact ttgtaac 27
<210> 21
   <211> 28
   <212> DNA
   <213> Artificial sequence
<220>
   <223> Reverse Primer for ENDO3
<400> 21
   agagtggtct tgggaatatt tatctcag 28
<210> 22
   <211> 26
   <212> DNA
   <213> Artificial sequence
<220>
   <223> Forward Primer for ENDO2
<400> 22
   acggatccca tttcaaagaa ctctga 26
<210> 23
   <211> 26
   <212> DNA
   <213> Artificial sequence
<220>
   <223> Reverse Primer for ENDO2
<400> 23
   gaccaatcat tatgctgtaa cttcag 26
<210> 24
   <211> 25
   <212> DNA
   <213> Artificial sequence
<220>
   <223> Forward Primer for ENDO1
<400> 24
   caggatccaa gtttcaaact tgaag 25
<210> 25
   <211> 26
   <212> DNA
   <213> Artificial sequence
<220>
   <223> Reverse Primer for ENDO1
<400> 25
   cggtatgtcg ggtttggttc aagtgg 26